# EUROPEAN PATENT SPECIFICATION

(11) **EP 4 295 293 B1**
(45) Date of publication and mention of the grant of the patent: **11.11.2026**
(21) Application number: 22706681.8
(22) Date of filing: 18.02.2022
(51) Int. Cl.: G06Q 10/08, G06Q 10/0832

(54) **A PROCESS AND AN INFRASTRUCTURE FOR MONITORING A LOAD OF PERISHABLE PRODUCTS**
VERFAHREN UND INFRASTRUKTUR ZUR ÜBERWACHUNG EINER LADUNG VERDERBLICHER PRODUKTE
PROCESSUS ET INFRASTRUCTURE DE SURVEILLANCE DE CHARGE DE PRODUITS PÉRISSABLES

(30) Priority: 22.02.2021 IT 202100004043
(43) Date of publication of application: 27.12.2023
(73) Proprietor: Cryovac, LLC, Charlotte, NC 28208 (US)
(72) Inventor: RIZZI, Jvanohe, 20025 Legnano (MI) (IT); FRANCINI, Giorgia, 20044 Arese (MI) (IT)
(74) Representative: PGA S.p.A., Milano, Succursale di Lugano
(86) International application number: PCT/IB2022/051471
(87) International publication number: WO 2022/175888

(56) References cited:
- WO-A1-2018/009646
- KR-B1- 102 187 438
- PAL AMITANGSHU AMITANGSHU PAL@TEMPLE EDU ET AL: "Smart Sensing, Communication, and Control in Perishable Food Supply Chain", ACM TRANSACTIONS ON SENSOR NETWORKS, ACM, 2 PENN PLAZA, SUITE 701 NEW YORK NY 10121-0701 USA, vol. 16, no. 1, 17 January 2020 (2020-01-17), pages 1 - 41, XP058468260, ISSN: 1550-4859, DOI: 10.1145/3360726

## Description

### FIELD OF THE INVENTION

The present invention refers to a process and an infrastructure for monitoring a load of perishable products. For example, the invention finds application in monitoring loads of perishable products such as foodstuffs from a production or sorting facility, where the products are packed and loaded onto a means of transport, during one or more transport stages to one or more destinations, where the products may be stored or displayed at a point of sale. The invention is particularly applicable to the monitoring of food products, fresh or frozen, but is generally applicable to any product likely to degrade over time: by way of example, pharmaceutical products, cosmetics, flowers, or industrial products that may degrade are included if, for example, subjected to inadequate thermal conditions.

### BACKGROUND ART

Various solutions are known that can monitor the storage conditions of products during their transport. For example, document WO2019/192741A1 concerns a monitoring system that uses 'blockchain' technology to certify that the quality of products remains adequate during transport. The system is designed to track products and detect product parameters that may have an impact on product quality. In particular, the sensors may include sensors attached directly to the product or its packaging and sensors positioned in the vehicle, which transmit measurement signals via radio to a monitoring control system. Document WO2019245717A1 concerns a monitoring system in which an indicator is attached to a perishable item or its packaging. The indicator includes ink marks that can be activated by a temperature change above a given threshold range. Activation causes an irreversible alteration of the temperature-sensitive ink marks providing a visible indication of previous temperature changes. In other words, once the indicator is activated and an aspect of the indicator alters to indicate the occurrence/detection of a temperature outside a threshold temperature range, the alteration is permanent and irreversible. A reader can then scan the indicators and inform a control network. A further known solution is described in US10521806B2, which describes a blockchain-based method for monitoring the product temperature during several stages of product life. The method involves the following steps:
- requirements for a product are received and stored by a smart label (e.g. RFID) fixed to a package containing the product; the requirements can be generated and stored in a block of the blockchain by a product manufacturer;
- the smart label also receives, from a temperature sensor, a temperature of the product (one or more temperature sensors can be placed inside and/or outside the package, and can be configured for transmitting temperature signals to the smart label);
- the product temperature is compared with a temperature or temperature range specified for the product saved in the smart label memory;
- the product temperature and the time the product temperature is received, are sent to the blockchain if the product temperature is outside a specified temperature range.

In practice, the smart label constantly receives the actual temperatures of the product from one or more sensors and compares the actual temperatures with the requirements. When the actual temperatures are greater or lower a specified temperature threshold, the actual temperatures and their corresponding recorded times can be transmitted to the blockchain.

In the known solutions described above, several types of sensors are therefore fixed to the product or its packaging and capable of sending information about the thermal condition of each product to a blockchain control system that supervises the status and therefore the quality of each product.

Although the solutions described are interesting, they are not free from drawbacks.

Indeed, the sensors typically used have relatively high costs and therefore the use of such technologies is not often suitable for use in all industries. In particular, in the food industry, packaged products have a relatively low cost that rarely justifies the use of technologies such as those described.

Moreover, managing the large amount of information from the sensors is often complex due to the large amount of data that is transmitted during monitoring and therefore must be sent and managed by a remote system. Finally, specifically in the field of distributed registers, there is the problem of ensuring that the data coming from the sensors are true and accurate.

A system for monitoring perishable products is described in WO2018009646A1. The known system has an environmental control assembly which controls the environmental parameters within a cargo compartment of a transport system. The known environmental control assembly comprises environmental parameter detectors for monitoring environmental parameters and product condition detectors for monitoring conditions of products located in the compartment. The known system comprises an analysis module adapted for receiving data from the environmental parameter detectors and product condition detectors for monitoring the products.

### OBJECT OF THE INVENTION

Object of the present invention is therefore to solve at least one of the drawbacks and/or limitations of the previous solutions.

A first object of the invention is to provide a process and an infrastructure for monitoring a load of perishable products, which can be implemented at relatively low cost and therefore has a negligible impact on the cost of the individual product.

It is also object of the present invention to provide a process and an infrastructure for monitoring one or more loads of perishable products by reducing the number of measurements and thereby reducing the amount of data to be transmitted and monitored.

It is object of the present invention to provide a process and an infrastructure for monitoring loads of perishable products which guarantees a high standard of reliability, so as to certify the quality of the monitored products in an indisputable manner.

It is a further object of the present invention to provide a process and an infrastructure for monitoring loads of perishable products which is well suited for monitoring the cold chain of food products, whether frozen or fresh. Finally, it is an object of the invention to provide a process and an infrastructure for monitoring loads of perishable products which can be installed relatively easily and which requires a small number of maintenance interventions.

These objects and others, which will become more apparent from the following description, are substantially achieved by a process and an infrastructure for monitoring a load of perishable products according to the claims.

### BRIEF DESCRIPTION OF THE DRAWINGS

Some embodiments and aspects of the invention will be described below with reference to the accompanying drawings, provided solely for illustrative purposes and therefore not-limiting purposes: wherein:
- Figure 1 is a block diagram relative to an infrastructure for monitoring a load of perishable products in accordance with the present invention;
- Figure 2 is a perspective view relating to a subgroup (e.g., a pallet) formed by a plurality of packages; the subgroup or pallet is housed within a loading bay served by the infrastructure according to the present invention;
- Figures 3-5 are schematic views of respective embodiments of an infrastructure according to the present invention;
- Figure 6 shows a flow chart of the steps of a process performed by an infrastructure in accordance with the present invention, in particular performed by the infrastructures of figures 3-5.

### CONTROL UNIT

The process and infrastructure for monitoring a load of perishable products described and claimed herein, respectively, make use of or comprise at least one control supervisor 6 and any further control units in charge of receiving signals from various sensors and performing one or more monitoring steps described and/or claimed.

The control unit in the supervisor 6 may be a single unit or may be formed by a plurality of distinct control units depending on the design choices and operational requirements.

N.B.: 'Circuit element' is a single active or passive functional part of an electronic circuit, such as one diode, one transistor, one resistor, one capacitor, etc. The term 'control unit' means an electronic component which may comprise at least one of: a digital processor (CPU), an analogue circuit, or a combination of one or more digital processors with one or more analogue circuits. The control unit may be "configured" or "programmed" to perform certain steps: this may be accomplished in practice by any means that allows the control unit to be configured or programmed. For example, in the case of a control unit comprising one or more CPUs and one or more memories, one or more programs may be stored in appropriate memory banks connected to the CPU(s); the program(s) contain instructions which, when executed by the CPU(s), program or configure the control unit to perform the operations described in relation to the control unit. Alternatively, if the control unit is or includes analogue type circuitry, then the circuitry of the control unit may be designed to include circuitry configured, in use, to process electrical signals such as to perform the steps related to the control unit.

### DETAILED DESCRIPTION

### Monitoring infrastructure

The term 1 has been generally indicated to describe an infrastructure for monitoring a load of perishable products, such as foodstuffs, pharmaceutical products or other products that are sensitive to the environmental conditions prevailing in the environment in which the product is stored; in particular, the infrastructure 1 may be used for monitoring products stored at a storage site or for monitoring products during transport from a production or sorting site to a destination site. The monitoring infrastructure 1 allows values representative of the state of preservation of the transported products to be measured and stored permanently within a distributed registry, for example implementing blockchain technology. The monitoring infrastructure 1 may be used, for example, in the transport of fresh or frozen foodstuffs, so as to certify that an ideal temperature is maintained during transport and that the quality of the products transported is guaranteed. In fact, the infrastructure makes it possible to monitor and certify the maintenance of the cold chain during the transport of fresh or frozen products, the latter typically kept at temperatures below 0°C. The monitoring infrastructure may also enable the implementation of smart contracts between a party responsible for the transport of perishable products and a recipient, in order to unequivocally define the responsibilities of the parties.

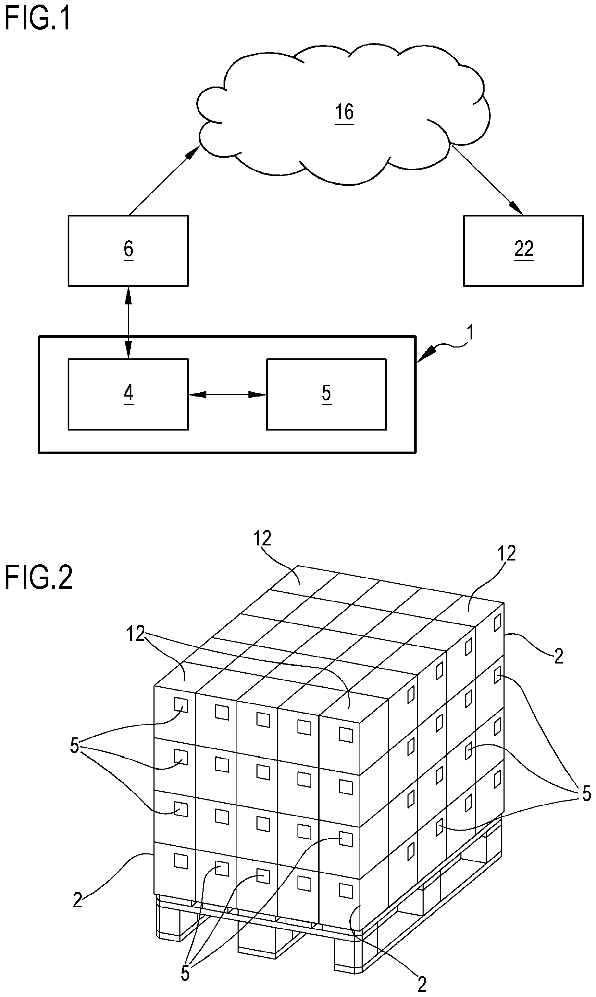

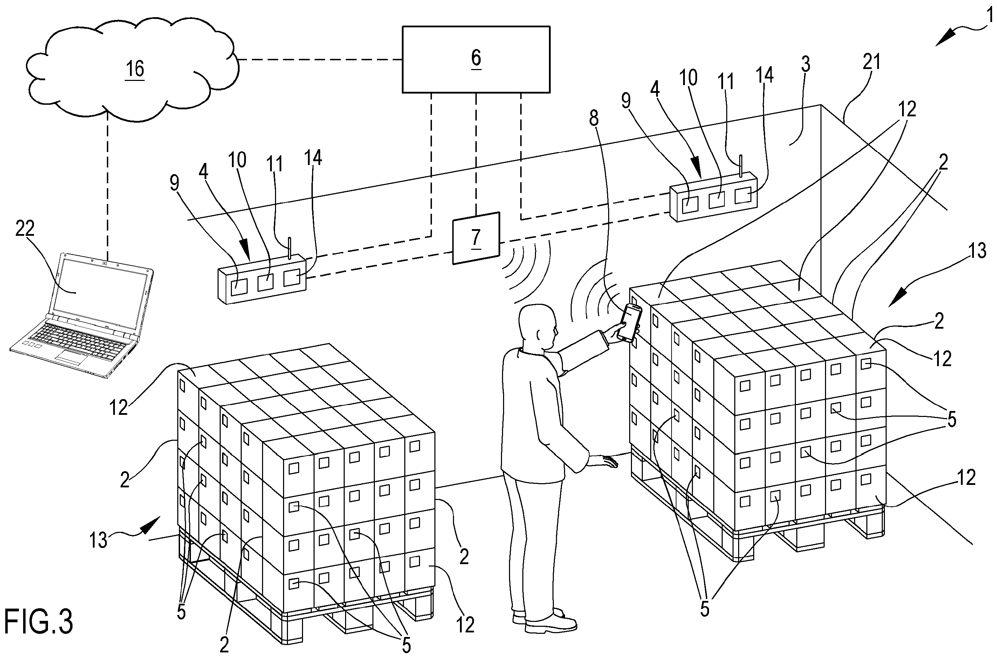

As for example visible in figures 3-5, the monitoring infrastructure 1 may be used to monitor product packages present in a load compartment 21 defined by a storage unit or conveyor. The load compartment 21 delimits a predetermined load volume 3 configured to house a plurality of packages 2 each of which houses one or more products to be transported. Note that the load volume 3 may be defined within a load volume 3 of a land vehicle, a ship, or an aircraft. In order to enable the infrastructure 1 to perform monitoring, the packages 2 are organized into a plurality of distinct subgroups 13 of adjacent packages in the load volume. Each subgroup 13 comprises a plurality of packages adjacent and superimposed each other, for example in such a way as to define pallets of cubic or parallelogram shape (figure 2).

Referring now to the example embodiment of figure 3, the infrastructure 1 comprises one or more first level sensors 4, operating in the load volume 21, configured to detect a first parameter related to an environment present in the load volume 3. In detail, Figure 3 involves the use of a plurality of first level sensors 4 distributed inside the load volume 3, such that they are sensitive to possible fluctuations of the first parameter in the various areas of the volume in which the sensors are installed. In fact, the load volume 3 may have a high spatial extension, making it preferable for the first parameter to be detected by the first level sensors at various positions within the load volume 3 itself. Each first level sensor 4 may comprise a memory 10 configured to store one or more values assumed by the first parameter, as well as to store a further parameter representative of the time instant at which each value of the first parameter is sampled. The memory 10 may further be configured to permanently store a predetermined unique identification code suitable for identifying each first level sensor 4 installed within the load volume 3.

The first level sensor 4 may further comprise a control unit 9 configured for executing a sampling of the first parameter and store, within the memory 10, the measured value of the first parameter and the value representative of the time instant at which sampling is performed. Note that the first parameter is representative of a temperature value or a parameter function of the temperature; or even of gases present in the environment or both of them. Note that specific gases are normally originated by products in their deterioration process (freshness markers), especially if the packaging used for the products is permeable or semi-permeable to gases. The first level sensor 4 may therefore comprise a temperature sensor or a sensor for the presence or concentration of a volatile substance, for example a gas sensor 14 configured to measure a temperature value within the load volume 3 at the position of the first level sensor 4. The first level sensor 4 is further configured to store within the memory 10, a pair of values, for example a temperature value or concentration of a gas/gas mixture present in the load volume 3 and an indicator of the sampling instant at which the temperature value or gas concentration is detected.

Below, in order not to make the discussion too heavy, we will describe the case in which the first parameter is the temperature, but as already mentioned, the concentration of a prefixed gas or gas mixture can alternatively be used as the first parameter and therefore what will be said below in relation to the temperature as the first parameter and the relative temperature values can be used, *mutatis mutandis,* apply to the case where the first parameter is the concentration or concentration variation measured by appropriate first sensors of a predetermined gas or gas mixture present in the environment of the load volume outside the packages.

Each first level sensor 4 may further comprise at least one transmitter 11 configured to send the pair of temperature-sampling instant values to a control supervisor 6, the latter detailed in the following. In particular, the transmitter 11 may for example be at least one of a Bluetooth transmitter, a WiFi transmitter, an RFID transponder tag, an NFC device. The transmitter 11 may be configured to send to the control supervisor 6 a package of information comprising the unique identification code of the first level sensor 4 and the pair of temperature values - sampling instant. The predetermined unique identification code allows each first level sensor 4 to be differentiated and consequently to associate each pair of temperature values -sampling instant with the first level sensor 4 that made the measurement.

The infrastructure may further comprise second level sensors 5: in particular, each second level sensor is associated with a respective package 2 and configured to detect an alteration of a second parameter relative to an environment adjacent to or inside each package 2. Each second level sensor 5 and thus each package are identifiable by a unique identification code suitable for distinguishing the packages 2 from each other. Each second level sensor comprises at least one component sensitive to the variation of the second parameter, the latter relative to a temperature value, or a parameter function of the temperature, of each package 2 or of an environment within each package. It should therefore be noted that the second level sensors may be suitable for detecting the instantaneous value of the measured parameter, or simply be sensitive to a variation or exceeding of a threshold relative to the parameter itself. In detail, each second level sensor 5 used in the example of figure 3 is suitable for sensing a variation of temperature inside or outside the package or a variation of another parameter providing the perishable state of the food product. In fact, the second level sensor 5 may be a sensor sensitive to:
- a temperature or parameter function of the temperature, carried on or in the product package,
- a component sensitive to the concentration of a predetermined gas inserted inside the package, or even outside in the case of permeable or semi-permeable packaging, or
- a pH-sensitive component of the product or other biochemical markers on its surface or in the environment inside the package.

Depending on the type of product, sensors sensitive to the most appropriate parameter can be used to indicate the state of deterioration of the product.

Since a large number of packages are present inside the load volume 3, and therefore a large number of second level sensors 5 (each of which is associated with a respective package 2), in order to keep a low cost of each package, the second level sensors 5 may be components of simple construction, for example also of the disposable type, and therefore of a different type than the first level sensors 4.

In particular, the second level sensors shown in figure 3 are applicable to the package 2, or can be inserted inside the package itself, or can be printed directly on the package 2 (for example on a plastic film or on a tray forming part of the package 2 itself).

Each second level sensor 5 can be configured to provide at least one between:
- a boolean indication of the fact that the product within the respective package 2 has exceeded or not a certain temperature for a specific time interval;
- an indication proportional to the maximum temperature reached by the product inside the respective package 2;
- a boolean indication of the fact that the area of the package 2 where the second level sensor 5 is present has exceeded or not a certain temperature for a specific time interval;
- an indication proportional to the maximum temperature reached by an area of the package 2 associated with the second level sensor 5;
- a boolean indication of the fact that the product inside the respective package 2 has exceeded or not a certain concentration of a predetermined gas over a specific time interval;
- an indication proportional to the maximum concentration of a predetermined gas reached by the product inside the respective packaging 2;
- a boolean indication of the fact that inside a respective package 2 a certain pH has been exceeded or not for a specific time interval;
- an indication proportional to the maximum or minimum pH reached inside the respective package 2;
- an indication that another indirect or direct marker of micro-organism growth, e.g. spoilage and/or pathogens, has reached a threshold within the package 2.

In practice, in the example of figure 3 each second level sensor 5 may be a passive sensor, unable to make measurements of absolute values of a parameter and storing the data obtained, but simply capable of providing an indication, for example visual, or a signal resulting from the detection of a change above a threshold of the monitored parameter. The second level sensor 5 may, for example, be a fluorimetric or colorimetric sensor capable of changing the coloration (or other visually appreciable property) of a surface of the same sensor as a function of temperature, so as to give evidence of a detection of a temperature (or a change thereof) on or in the package above a predetermined threshold. Alternatively, the second level sensor 5 may modify a graphical portion, which may for example comprise a QR code or barcode, so as to identify the exceeding of a threshold temperature or a predetermined thermal variation. In a further variant, the second level sensor 5 may comprise a magnetic ink print, made on the tray or film of the package: when subject to a magnetic field, the sensor is able to emit a magnetic signal proportional to the temperature, which thus allows indirect measurement of the temperature value on or in the package.

Although the parameter monitored in the context of food products is often the temperature, the possibility of monitoring parameters such as the concentration of a substance or gas or the degree of acidity inside the package with second level sensors is not excluded (as already mentioned), preferably using passive elements of simple design that can provide an indication or signal related to the monitored parameter.

The infrastructure of figure 3 comprises at least one detector 8 configured to read and/or receive information emitted by the second level sensors 5. The detector 8 may be a dedicated device for reading the information emitted by the second level sensors 5, such as for example an optical scanner or a camera, an electromagnetic wave detector or an acoustic detector. However, it is not excluded that the detector 8 may comprise an application (in particular software program) installable on a general purpose device, such as a smartphone or tablet, capable when executed of configuring the general purpose device to read or receive the indications and/or signals displayed and/or emitted by the second level sensors.

The detector(s) 8 is (are) communicatively connected to the first level sensors 4 (which in this case act as intermediaries) or directly connected to the control supervisor 6.

The detector 8 can effectively detect and analyze the information generated/visualized by the second level sensors, store it in a local memory and transmit it to the control supervisor 6.

In accordance with the first embodiment of the invention shown in Figure 3, the detector 8 is a device manually operated by an operator interrogating the second level sensors 5. The analog information (for example, visual, magnetic or acoustic) emitted/shown by the second level sensors is, via the detector 8, transformed into information, for example digital, that can be transmitted to one or more other components of the infrastructure. The detector 8 may further have a user interface suitable for indicating to the person in charge, the unique identification code of the second level sensors 5 to be interrogated. The first level sensors 4 or the control supervisor 6, can be configured to communicate to the detector 8, the reading of predetermined second level sensors 5 according to a predetermined criterion, in such a way as to optimize the timing of reading the information as well as to impose the sensors to be consulted, useful to certify the quality of the entire pallet without the need to interrogate all the second level sensors of the pallet itself.

The infrastructure may further comprise an intermediate transmission unit 7 communicatively interposed between the first level sensors 4 and the control supervisor 6. In detail, the intermediate transmission unit 7 may be defined by a digital and/or an analogue processing unit suitable for defining a data transmission channel between the first level sensors 4 and the control supervisor 6. The intermediate transmission unit 7 may for example comprise a router, suitable for defining a local network configured for putting each first level sensor 4 into communication with the control supervisor 6, or to define communication between the latter via the internet network.

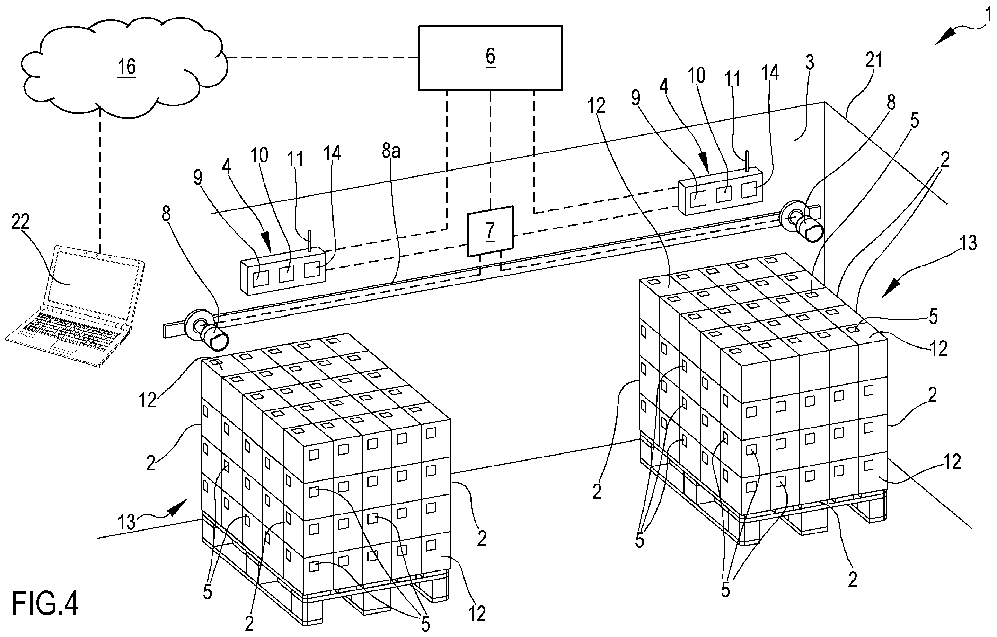

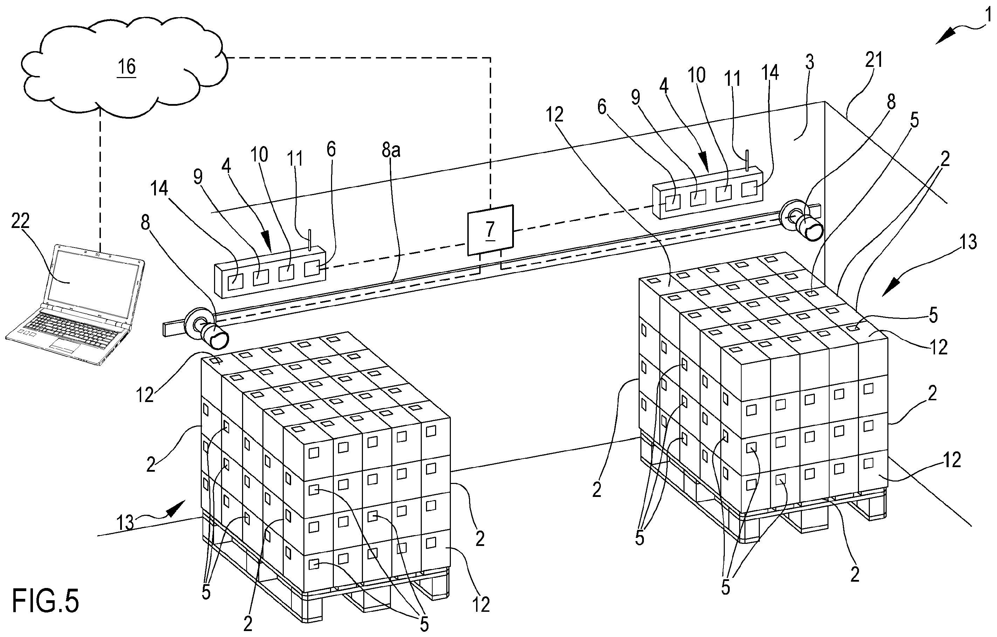

As previously mentioned, the infrastructure may comprise at least one control supervisor 6 formed by a digital and/or analog processing unit directly or indirectly connected to the first level sensors 4, the detector 8 and possibly also to the second level sensors 5. The control supervisor is configured for commanding the execution of the process that will be described later and to receive from the first sensors the values of the first parameter and from the detector or the second sensors the indications/values relative to the second parameter. In other words, the control supervisor 6 can be defined as a control unit suitable for coordinating the first level sensors 4 and the detector 8, respectively for measuring the first and the second parameter. From a structural point of view (figure 3 and figure 4), the control supervisor 6 is arranged in a remote position with respect to the load volume 3. However, it is not excluded that the control supervisor 6 is arranged inside the load volume 3, or included within each first level sensor 4 (as will be illustrated in the example in accordance with the third embodiment of the infrastructure shown in figure 5).

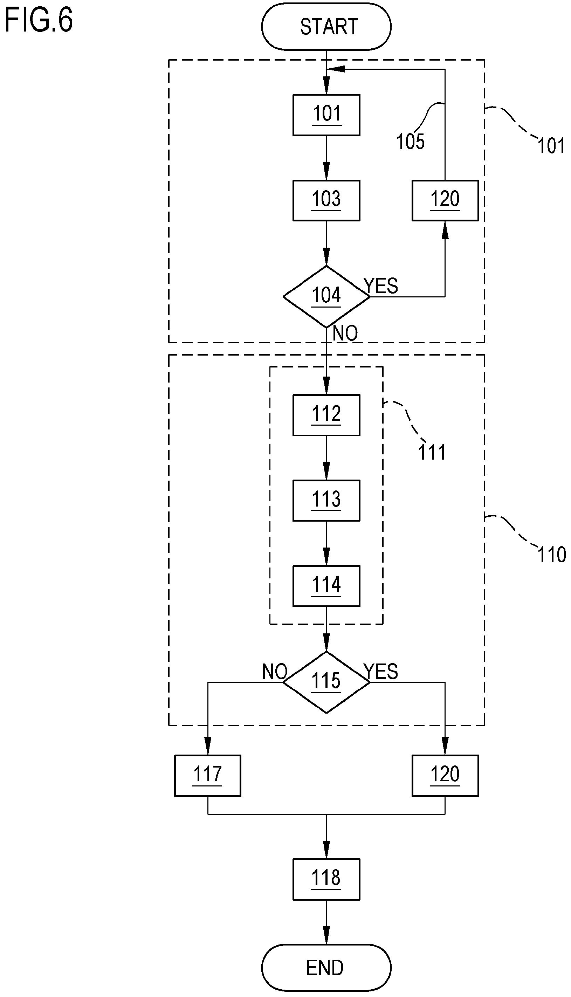

The control supervisor 6 is configured to execute a monitoring process 100 (see figure 6) comprising the execution of a first level procedure 101 and a subordinate second level procedure 110 which act in cascade upon the occurrence of the conditions described below. In particular, the monitoring supervisor 6 is configured to primarily execute the first level procedure 101 comprising the steps of:
- commanding each first level sensor 4 to sample the first parameter (step 102),
- receiving, or determine, on the basis of the signals emitted by each first level sensor 4, the values assumed by the first parameter (step 103),
- determining if the values of the first parameter comply or not with a predetermined first level acceptability criterion (step 104).

If, as a result of the query referred to in step 104, it is determined by the control supervisor 6 that the values of the first parameter comply with the predetermined first level acceptability criterion, the procedure comprises cyclically repeating the execution of the first level procedure (see recirculation line 105 in figure 6). For example, the supervisor may be configured to repeat the execution of steps 102-104 periodically (for example after a time interval of one or more minutes has elapsed), or upon command of an input given by an operator suitable for communicating with the supervisor, or following the occurrence of a predetermined event (for example the variation of an external parameter, the running of a predetermined kilometric route or other event), or at regular intervals given by a predetermined rule managed by the supervisor (for example the repetition of the cycle may occur more frequently as time goes by).

As previously mentioned, the control supervisor 6 is configured to receive from each first level sensor 4 the aforementioned package of information (for example comprising the unique identification code of the first level sensor 4 and the pair temperature value sampling time instant), so as to determine whether the sampled first parameter values comply with the first level acceptability criterion or not (step 104). In fact, the comparison with the first level acceptability criterion may comprise comparing each first parameter value sampled by each sensor 4 with a range of values deemed acceptable, for example a range of temperatures, or a range of parameter values as a function of temperature.

If the first level acceptability criterion is not complied with, the control supervisor 6 is configured to perform the second level procedure (step 110). In particular, the supervisor may connect communicatively with the detector 8, so as to receive information relative to the second parameter provided by the second level sensors 5.

In particular, the second level procedure (step 110) allows to identify and report subgroups 13 of packages 2 that do not comply with a prefixed second level acceptability criterion on the basis of the information provided by the second level sensors 5. In particular, the prefixed second level acceptability criterion is considered to be complied with for a given subgroup 13, only if the information provided by each of the second level sensors 5 belonging to the subgroup under examination, responds to a respective verification test.

The second level procedure thus allows to determine which subgroups 13 comply with a predetermined second level acceptability criterion. In particular, the second level procedure allows to identify for each subgroup 13, a prefixed number of sample packages 12, as well as to establish if a subgroup 13 complies or not with the predetermined second level acceptability criterion exclusively using the information emitted by the second level sensors 5 exclusively associated with the sample packages 12. In this regard, it should be noted that each subgroup 13 belongs to a predetermined number of sample packages 12, which are smaller in number than the total number of packages of the subgroup 13 to which it belongs. In particular, the sample packages 12 are preferably the packages placed in an outer peripheral area of the subgroup itself, for example at a respective edge or a respective upper or lower vertex of the pallet.

It should be noted that the analysis of the sample 12 packages alone minimizes the time required to carry out the second level procedure and reduces the amount of information collected to manage the second level verification test. As a matter of fact, the packages 2 placed at the top of the pallet are the packages most subject to heat loss (or to the action of external agents of any nature), the analysis of the sample packages 12 only allows therefore to measure the temperature value or to detect a temperature variation (or other parameter) in correspondence with the packages that have been subject the greatest thermal change (or change in other monitored parameter). In other words, verifying that the temperature readings (or other parameter) provided by the second level sensors 5 associated with the sample packages 12 comply with the predetermined second level acceptability criterion ensures that the predetermined second level acceptability criterion is also complied with for the untested packages.

More in detail, still referring to the flow chart of figure 6, the second level procedure involves identifying in each subgroup 13 the sample packages 12 to be analyzed by executing an interrogation protocol (step 111). In particular, the control supervisor 6 is configured to select, as sample packages 12, those belonging to a list of unique identification codes of the packages 2 present in the interrogation protocol (step 112), for example corresponding to the packages 2 arranged at the vertices of the pallet.

The control supervisor 6 is then configured to send the command signal to each of the detectors 8 to command the latter to read the second level sensors according to the interrogation protocol (always step 112). The control supervisor 6 is further configured to receive directly from the second level sensors or the detector 8 the indication relative to the second parameter detected by the second sensors 5, as well as to receive the respective unique identification code of the respective package 2 of each of the queried second sensors 5 (step 113). The control supervisor 6 is configured to verify that the sample packages 12 identified in each subgroup 13 have an identification code that is the same as the sample packages 12 in the interrogation protocol list (step 114). If there is a match between said identification codes, the data is considered valid, otherwise the control supervisor 6 sends a new command signal to the detector 8 to request the re-reading of a second level sensor 5 present in the interrogation protocol.

Following receipt of the information sampled by the second level sensors 5 and verification that this information has been recorded by the second level sensors 5 belonging to the querying protocol, the control supervisor 6 is configured for executing the verification test to determine whether the data sampled by the second level sensors comply with the second level acceptability criterion (step 115). As discussed above, the supervisor 6 is then configured for comparing each information/value of the second sampled parameter with a respective acceptability criterion (part of step 115), for example in case the parameter of interest is temperature with an acceptable temperature range, or a reference (chromatic, acoustic, or other nature) deemed acceptable.

Depending on the information detected by the first and second level sensors 4, 5, the control supervisor 6 is configured for certifying that one or more subgroups 13 present in the load volume 3 are formed exclusively of products that comply with a predetermined quality standard (i.e. from a level of quality deemed acceptable). In other words, for example during transport of the load from a delivery point to a destination, the control supervisor 6 certifies the acceptable quality of all products transported (step 120) if, in a reference time interval during which the first level procedure is performed a plurality of times, the following is verified:
- the values of the first parameter comply with the predetermined first level acceptability criterion each time the first level procedure is performed,
   or if
- the values of the first parameter do not comply with the predetermined first level acceptability criterion during at least one execution of the first level procedure, and
- when the second level procedure is performed, it is verified that said one or more subgroups (13) comply with the predetermined second level acceptability criterion.

In practice, the reference time interval may coincide with the duration of a load transport from a delivery point to a destination: during this interval the monitoring process is carried out by repeating the first level procedure several times. If the first level acceptability criterion is always complied with each time the first level procedure is performed, it goes without saying that the quality can be certified without the need to perform the second level procedure. If, on the contrary, one or more times the first level procedure is not positively passed (first acceptability criterion not satisfied), the second level procedure is activated: after the execution of the second level procedure for each subgroup or pallet, the compliance with the second acceptability criterion is verified for all the subgroups present in load volume 3 (step 115). If the second acceptability criterion is complied with for all the subgroups, the monitoring process requires the inspection supervisor to certify the acceptable quality of all the products present in the loading volume itself (step 120). The control supervisor is configured for emitting a signal indicating that one or more subgroups or pallets do not comply with the intended acceptability criterion (step 117) and therefore it is not possible for those subgroups to certify acceptable quality.

The supervisor 6 is further configured to communicate periodically or at regular intervals or following a command or following the detection of an event, to a blockchain-type distributed registry 16 connected to the control supervisor 6, at least one (preferably all) of the following parameters (step 118):
- the unique identification code relating to each first level sensor 4 operating in load volume 3,
- the pair temperature values (or value of other first parameter detected by the first level sensors)-sampling time instant measured by each first level sensor 4 operating in the load volume 3 during the execution of the first level procedure,
- the unique identification code of a second level sensor 5 operating in load volume 3,
- the pair temperature value (or other indication given by the second level sensors)-detection time instant recorded by the detector 8.

Each of the data detected and/or measured by the first and second level sensors 4, 5, whether or not they respectively comply with the first and second level acceptability criteria, are available to a user of the infrastructure exclusively following access to the distributed register via a user device connected to a local area network or to an internet network. The infrastructure allows a user to download each of the data respectively measured and/or detected by the first level sensor 4 and the detector 8, so as to recreate the thermal history (or the history of any other parameter or set of parameters) of the transported products. The use of the blockchain technology allows to give evidence to the user of possible tampering of the temperature data and/or sampling instants stored in the distributed register, guaranteeing the genuineness of the detected and stored data and consequently of the transported product.

The infrastructure may further comprise at least one emitter 22 of a visual and/or acoustic signal arranged at the side of the load compartment or remotely, connected to the distributed register 16 and configured for giving evidence to a user of non-compliance with the second level acceptability criterion for a predetermined time interval. Note that the emitter 22, in an embodiment of the invention is an application for example installable on a smartphone or a PC, suitable for notifying a subject receiving the load, of non-compliance with the second level acceptability criterion.

In the embodiment exemplified in figure 4, an infrastructure 1 is schematically illustrated having most of the structural and functional characteristics described above and also suitable for executing the monitoring process schematically illustrated in figure 6. In order to avoid repetitions, in figure 4 the components already described with reference to the example of figure 3 have been represented with the same numerical references.

In particular, the second embodiment shown in figure 4, provides that the detector 8 may comprise at least one of a camera and a radio wave detector, configured for automatically reading the information shown/supplied by the second level sensors 5. In particular, the detector 8 may have a plurality of cameras and/or radio wave detectors distributed throughout the load volume 3 and facing the packages 2, so as to easily read the information shown/supplied by the second sensors 5, digitize it and transmit it to the control supervisor 6 or to a first level sensor 4. Each camera or radio wave detector, may further be movable along rails 8a integral with the loading area 21, in such a way as to scan at least part of the load volume 3. Each detector 8 may further have a respective control unit connected to the first level sensors 4 and/or the control supervisor 6 and configured for receiving a command signal emitted by a first level sensor 4 or by the control supervisor 6, representative of the position inside the load volume 3, of the second level sensors 5 to be read. Each detector 8, following receipt of the command signal, is configured for moving relative to the load volume 3, along the guides 8a and position itself at a second level sensor 5 to be read. The detector 8 is then configured for transmitting to the first level sensor 4 or the supervisor 6 the detected information.

According to the latter embodiment of the invention, a reduced number of cameras and/or radio detectors allow for the reading of the second level 5 sensors in an automatic manner, thereby decreasing the manufacturing costs of the infrastructure.

In the example embodiment of figure 5, an infrastructure 1 having the structural and functional characteristics described above with reference to figure 4 and also suitable for executing the monitoring process schematically illustrated in figure 6 is schematically illustrated. In order to avoid repetitions, in figure 5 the components already described with reference to the embodiment of figure 3 and figure 4 have been represented with the same numerical references. The only difference with respect to the embodiment of figure 4 concerns the control supervisor 6 which in figure 5 is incorporated in one or more of the first level sensors. In this embodiment, therefore, the supervisor hosted by the first level sensor interacts with the other first level sensors and with the second level sensors by performing the process of figure 6.

The described and claimed process and infrastructure achieves one or more of the stated objectives and also guarantees a high degree of reliability through a redundancy of detection levels, which in each case is carried out with a criterion that minimizes the complexity of the system and the data load to be managed.

The process and infrastructure described and claimed herein may comprise further embodiments falling within the scope of protection of the accompanying claims. In particular, although the process described and claimed comprises the execution of a first level procedure (using the first level sensors) and a second level procedure (using the second level sensors), it may be contemplated that the process further comprises a third level procedure (or further levels of inspection) which is executed following the execution of the second level procedure to analyze even more extensively subgroups of subgroups (if, for example, dealing with loads involving a large number of products). Similarly, the infrastructure 1 may be configured for executing a process comprising a first level procedure (using the first level sensors) and a second level procedure (using the second level sensors) and a third level procedure (as well as further inspection levels) which is executed following the execution of the second level procedure to analyze even more capillary subgroups of subgroups (if, for example, dealing with loads involving a large number of products). In addition, each of the first level sensors may comprise several sensors combining, in a single unit suitable for providing the reading of various first level parameters, the different types of first level sensors provided above. Similarly, each of the second level sensors may comprise multiple sensors combining the different types of second level sensors provided above thus being able to provide various types of information for each package.

Finally, from a physical point of view, the control supervisor, as already mentioned, may be a remote unit with respect to the load compartment or alternatively it may include one or more units in the load compartment. In particular (according to the desired implementation architecture) the control supervisor may partly comprise elements arranged in the load compartment and partly elements remote from the same: in fact the control supervisor, performing calculation functions, may be centralized or not and therefore located entirely or partly in the load compartment or in proximity thereto (for example on board the means for transportation where the load compartment is located). In a particular alternative, part of the supervisor's computing functions could be incorporated into the detector or into a smartphone or tablet or other portable device that also includes the detector 8.

## Claims

1. Infrastructure for monitoring a load of perishable products, said load comprising a multiplicity of products housed in respective packages (2) and placed in a load volume (3) of a storage unit or of a conveyor, wherein:
- the load volume (3) is provided with a predetermined number of first level sensors (4) configured for detecting a first parameter relative to an environment present in the load volume itself,
- the load volume (3) comprises a plurality of subgroups (13) of said multiplicity of products present in the load volume itself, wherein each subgroup in turn comprises a plurality of products which are, in number, a fraction and in particular submultiple of said multiplicity of products present in the load volume,
- each of the packages (2) or of the products housed in said packages is associated with at least one second level sensor (5), each second level sensor (5) being sensitive at least to an alteration of a second parameter relative to an environment adjacent to or inside each package;
said infrastructure (1) comprising at least one control supervisor (6) configured for executing a first level procedure which comprises the following steps:
- receiving, or determining based on signals emitted by the first level sensors, values assumed by said first parameter over time based on detections of the first parameter carried out by said predetermined number of first level sensors (4),
- establishing if said values of the first parameter comply with or do not comply with a predetermined first level acceptability criterion,
- if the values of the first parameter comply with the pre-established first level acceptability criterion, repeating the execution of the first level procedure,
said infrastructure also being configured for executing a second level procedure if the values of the first parameter do not comply with the predetermined first level acceptability criterion, the second level procedure comprising the following steps:
- establishing which subgroups (13) comply with a predetermined second level acceptability criterion, using indications relative to the alteration of the second parameter detected by at least one part of the second level sensors (5) associated with the products or with the packages (2) present in each respective subgroup.

2. Monitoring infrastructure according to claim 1, wherein the second level sensors (5) are of type different from that of the first level sensors (4),
wherein the control supervisor is configured for executing the second level procedure, and
wherein during execution of the second level procedure the control supervisor (6) is configured for:
- being communicatively connected with the first level sensors (4) or with an intermediate transmission unit (7) communicatively interposed between the control supervisor (6) and the first level sensors (4), and
- receiving the signals emitted by the first level sensors (4) or directly the values assumed by the first parameter over time;
and for:
- being communicatively connected with the second level sensors (5) or with a detector (8), configured for reading the indications emitted by the second level sensors (5), communicatively interposed between the control supervisor (6) and the second level sensors (5) and
- receiving, from said detector (8) or directly from said second level sensors (5), the indications relative to the alteration of the second parameter;
in particular wherein the control supervisor (6) is housed in the load volume (3) or in remote position with respect to the load volume.

3. Monitoring infrastructure according to claim 1 or 2, wherein the control supervisor (6) is configured for executing the further step of certifying that the products in the load volume (3) all comply with a predetermined quality standard if, in a reference time interval during which the first level procedure is executed a plurality of times, the following is verified:
- the values of the first parameter comply with the predetermined first level acceptability criterion during each execution of the first level procedure, or
- the second level procedure is never executed, or
- if the second level procedure is executed, it is verified that all the subgroups comply with the predetermined second level acceptability criterion;
optionally wherein the reference time interval is equal to the duration of a transport of a load from a delivery point to a destination.

4. Monitoring infrastructure according to any one of the preceding claims comprising the first level sensors, wherein each of the first level sensors (4) comprises a respective control unit (9), a memory (10) and a transmitter (11), communicatively connected to each other in a direct or indirect manner, and is configured for:
- storing one or more values assumed over time by the first parameter and
- transmitting such one or more values to the control supervisor configured for executing the first level procedure;
optionally wherein each of said first level sensors (4) comprises a Bluetooth transmitter, a WiFi transmitter, a RFID transponder tag, an NFC, and
wherein the first parameter is detected at the position in the load volume in which each first level sensor (4) operates and is one of the following:
- the temperature, or
- a parameter that is a function of the temperature, or
- the concentration of a predetermined gas or gas mixture, or
- the variation of concentration of a predetermined gas or gas mixture,
wherein said predetermined first level acceptability criterion is respectively defined by
- a temperature interval, or
- an interval of values of the parameter that is a function of the temperature, or
- a concentration interval of a predetermined gas or gas mixture, or
- an interval in the concentration variation of a predetermined gas or gas mixture, and
wherein a plurality of first level sensors (4) are distributed, optionally uniformly distributed, in the load volume (3), each of the first level sensors (4) being associated with a unique identification code;
wherein the first level sensors (4) are configured for transmitting, to the control supervisor (6), the values assumed over time by the first parameter associating the respective unique identification code of each of the first sensors, and
wherein the control supervisor (6) is configured for identifying the values assumed over time by the first parameter detected by each of said first sensors.

5. Monitoring infrastructure according to any one of the preceding claims, wherein the second parameter is one of the following:
- the temperature of an environment inside the package (2), or
- a parameter that is a function of the temperature of an environment inside the package (2), or
- the temperature of an external surface of the package (2),
- a parameter that is a function of the temperature of an external surface of the package (2),
- the concentration of a predetermined gas or of a predetermined substance inside the package (2),
- the concentration variation of a predetermined gas or substance inside the package, or
- the concentration of a predetermined gas or of a predetermined substance on an external surface of the package (2),
- the concentration variation of a predetermined gas or substance on an external surface of the package, or
- the degree of acidity present inside the package (2) or
- the variation of the degree of acidity inside the package (2), and
wherein each of the second level sensors (5) associated with a respective package comprises at least one of the following:
- a component sensitive to the temperature or to a parameter proportional to the temperature carried by the package of the product,
- a component sensitive to the temperature or to a parameter proportional to the temperature inserted inside the package,
- a component, sensitive to the concentration or to the concentration variation of a predetermined gas, inserted inside the package,
- a component, sensitive to the concentration of a predetermined substance or to the variation of a predetermined substance, inserted inside the package,
- a component, sensitive to the concentration or to the concentration variation of a predetermined gas, carried by the package,
- a component, sensitive to the concentration of a predetermined substance or to the variation of a predetermined substance, carried by the package,
- a component sensitive to the pH or to the pH variation of the environment inside the package;
optionally wherein each of the second level sensors (5) associated with a respective package (2) is
- a disposable component applied to the package (2) or inserted in the package, or
- a component printed directly on the package (2), optionally on a plastic film or on a plastic or paper tray which is part of the package (2).

6. Monitoring infrastructure according to any one of the preceding claims, wherein each of the second level sensors (5) is configured for providing at least one of the following:
- an indication, optionally visual, of yes/no type of the fact that the product inside the respective package has exceeded or not exceeded a certain temperature for a specific time interval;
- an indication, optionally visual, proportional to the maximum temperature reached by the product inside the respective package;
- an indication, optionally visual, of yes/no type of the fact that the zone of the package where the second level sensor is present has exceeded or not exceeded a certain temperature for a specific time interval;
- an indication, optionally visual, proportional to the maximum temperature reached in the zone of the package where the second level sensor is present;
- an indication, optionally visual, of yes/no type of the fact that inside the respective package, a certain concentration or variation of concentration of a predetermined gas or gas mixture has been exceeded or not exceeded for a specific time interval;
- an indication, optionally visual, proportional to the maximum concentration of a predetermined gas or gas mixture reached inside the respective package;
- an indication, optionally visual, of yes/no type of the fact that inside the respective package, a certain concentration or variation of concentration of a predetermined substance or group of substances has been exceeded or not exceeded for a specific time interval;
- an indication, optionally visual, proportional to the maximum concentration of a predetermined substance or group of substances inside the respective package;
- an indication, optionally visual, of yes/no type of the fact that at a surface of the respective package a certain concentration or variation of concentration of a predetermined gas or gas mixture has been exceeded or not exceeded for a specific time interval;
- an indication, optionally visual, proportional to the maximum concentration of a predetermined gas or gas mixture reached at a surface of the respective package;
- an indication, optionally visual, of yes/no type of the fact that inside the respective package a certain pH or a certain variation of pH has been exceeded or not exceeded for a specific time interval;
- an indication, optionally visual, proportional to the maximum or minimum pH reached inside the respective package;
wherein, the predetermined second level acceptability criterion is considered complied with for a specific subgroup only if the indication, optionally visual, provided by each of the second level sensors (5) used in the second level procedure for such subgroup meets a respective verification test.

7. Monitoring infrastructure according to any one of the preceding claims, wherein the step of the second level procedure of establishing which subgroups comply with a predetermined second level acceptability criterion comprises:
- receiving an identification, for each subgroup (13), of a predetermined number of sample packages (12), said sample packages (12) being in a number lower than the total number of packages in each subgroup (13), in particular wherein said sample packages are in a number lower than half the packages (2) present in each subgroup (13),
- establishing that a subgroup complies with the predetermined second level acceptability criterion by only using the second level sensors (5) associated with the sample packages (12), or with the products contained in the sample packages (12), present in the respective subgroup (13);
wherein the sample packages (12) of each subgroup (13) are placed in an external peripheral zone of the subgroup itself;
optionally wherein when each subgroup (13) has packages (2) adjacent and superimposed on each other to form a cube or an ideal parallelogram, each of the sample packages (12) is placed at a respective edge or a respective vertex of the cube or ideal parallelogram,
more optionally wherein the sample packages (12) are two or more, still more optionally from 3 to 6, for each subgroup (13), and
wherein each package (2), in particular each of the second level sensors (5) of each package (2), is associated with a unique identification code, and wherein the step of establishing which subgroups comply with a predetermined second level acceptability criterion comprises, for each subgroup:
- receiving directly, or from said detector (8), at least one indication relative to the second parameter, emitted or displayed by each of the interrogated or observed second sensors (5),
- receiving, in association with each indication, the respective unique identification code of the respective package (2) of each of the interrogated or observed second sensors (5).

8. Monitoring infrastructure according to claim 7, wherein the control supervisor (6) or the detector (8) is configured for identifying, in each subgroup (13), the sample packages (12) by using an interrogation protocol that comprises a list of unique identification codes of the packages (2), and wherein the control supervisor (6) or the detector (8) is configured for selecting as sample packages (12) those belonging to the list present in the interrogation protocol, and
wherein the second level procedure comprises a verification step, carried out directly by the control supervisor (6) or by the detector (8), in which the sample packages (12) identified in each subgroup (13) are all and only those present in the or in a list of the of an interrogation protocol comprising the unique identification codes of the sample packages (12) to be selected, optionally wherein the interrogation protocol is stored in the control supervisor (6).

9. Monitoring infrastructure according to any one of the claims 2 to 8, wherein said indication provided by each of the second level sensors (5) is read by the detector (8) communicatively connected with the control supervisor (6) and is transmitted to the latter, in particular wherein the detector comprises:
- at least one optical scanner or a video camera if the indication is of visual type, or
- at least one radio wave detector, if the indication is of electromagnetic type, or
- at least one acoustic detector, if the indication is of sound type.

10. Monitoring infrastructure according to any one of the preceding claims, wherein the control supervisor comprises one or more digital computers and/or one or more analog computers and wherein the control supervisor (6) is configured for communicating, periodically or at regular intervals or following a command or following the detection of an event, to a distributed register (16) of blockchain type connected to said control supervisor (6), at least one or more, optionally all, of the following:
- the unique identification code relative to each first level sensor (4),
- the pair of values, formed by a value of the first parameter and sampling instant, detected by each first level sensor (4) during the execution of the first level procedure,
- the unique identification code of each second level sensor (5),
- the pair of values, formed by an indication relative to the second parameter and detection instant, recorded by the detector (8) for each interrogated second level sensor, and
wherein the control supervisor (6) is configured for certifying which one or more subgroups (13) present in the load volume (3) are only formed by products which comply with a predetermined quality standard if, in a reference time interval during which the first level procedure is executed a plurality of times, is verified as follows:
- the values of the first parameter comply with the predetermined first level acceptability criterion upon each execution of the first level procedure,
or if
- the values of the first parameter do not comply with the predetermined first level acceptability criterion during at least one execution of the first level procedure, and
- once the second level procedure is executed, it is verified that said one or more subgroups (13) comply with the predetermined second level acceptability criterion;
optionally wherein the reference time interval is equal to the duration of a transport of the load from a delivery point to a destination.

11. A computer implemented process of monitoring a load of perishable products, said load comprising a multiplicity of products housed in respective packages (2) and placed in a load volume of a storage unit or a conveyor, wherein:
- the load volume (3) is provided with a predetermined number of first-level sensors (4) configured for detecting a first parameter relative to an environment present in the load volume (3) itself,
- the load volume (3) comprises a plurality of subgroups (13) of said multiplicity of products present in that load volume, wherein each subgroup (13) comprises a plurality of products which are, in number, a fraction, and in particular a submultiple of said multiplicity of products present in the load volume (3),
- each of the packages (2) or of the products housed in said packages (2) is associated with at least one second level sensor (5), each second level sensor (5) being sensitive to at least one alteration of a second parameter relative to each package (2) or to an environment inside each package (2);
wherein said process includes the execution of a first level procedure comprising the following steps:
- determining values assumed by said first parameter over time based on detections of said first parameter carried out by said predetermined number of first level sensors (4),
- establishing if said values of the first parameter comply with or do not comply with a predetermined first level acceptability criterion,
- if the values of the first parameter comply with the predetermined first-level acceptability criterion, repeating the execution of the first level procedure;
and wherein said process includes a second level procedure which is performed if the values of the first parameter do not comply with the predetermined first level acceptability criterion, the second level procedure comprising the following steps:
- establishing which subgroups (13) comply with a predetermined second level acceptability criterion, using indications relative to the alteration of the second parameter detected by at least one part of the second level sensors (5) associated with the products or with the packages (2) present in each respective subgroup.

12. The process of claim 11, wherein the second level sensors (5) are of type different from that of the first level sensors (4), and/or
wherein the load volume (3) comprises a plurality of subgroups (13) of products, each subgroup (13) being identifiable and distinct from the other subgroups, in particular wherein each subgroup is housed in a respective enclosure or placed on a respective pallet, and/or
wherein the process comprises the further step of certifying that all the products in the load volume (3) comply with a predetermined quality standard if, in a reference time interval during which the first level procedure is performed a plurality of times, the following is verified:
- the values of the first parameter comply with the predetermined first level acceptability criterion during each execution of the first level procedure;
optionally the reference time being equal to the duration of a transport of a load from a delivery point to a destination.

13. The process of any one of the preceding two claims, wherein the process comprises the further step of certifying that the products in the load volume (3) all meet a predetermined quality standard if, in a reference time interval during which the first level procedure is performed a plurality of times, the following is verified:
- the second level procedure is never performed;
and wherein the process comprises the further step of certifying that all the products in the load volume (3) comply with a predetermined quality standard if, in a reference time interval during which the first level procedure is performed a plurality of times, the following occurs:
- if the second level procedure is performed, it is verified that all subgroups comply with the predetermined second level acceptability criterion, and
wherein the second level procedure includes the additional step of reporting subgroups that do not comply with the predetermined second level acceptability criterion.

14. The process of any one of claims 11-13, wherein the first parameter is detected at the position inside the load volume in which each first level sensor (4) operates; in particular wherein a plurality of first level sensors (4) are distributed, optionally uniformly distributed, in the load volume, and
wherein the predetermined first level acceptability criterion is defined by a range of temperatures, or by a range of parameter acceptability values as a function of temperature or temperature variation;
or wherein the predetermined first level acceptability criterion is defined by a concentration range or concentration variation of a prefixed gas or gas mixture, and
wherein the second parameter is the temperature, or a temperature-dependent parameter, of each package (2) or of an environment inside each package; or the second parameter is the temperature of an outer surface of the package (2); or the second parameter is the temperature inside each package (2): or the second parameter is the concentration or concentration variation of a predetermined gas inside the package (2); or the second parameter is the concentration or concentration variation of a predetermined substance inside the package; or the second parameter is the degree of acidity present inside the package (2) or the variation of the degree of acidity in each package (2).

15. The process of any one of claims 11-14, wherein each of the second level sensors (5) provides that the predetermined second level acceptability criterion is considered to be complied with for a given subgroup (13) only if the indication, optionally visual, provided by each of the second level sensors (5) used in the second level procedure for that subgroup complies with a respective verification test, and
wherein the step of the second level procedure of determining which subgroups comply with a predetermined second level acceptability criterion includes:
- identifying in each subgroup (13) a predetermined number of sample packs (12), said sample packs (12) being smaller in number than the total number of packs (2) in each subgroup (13), in particular where said sample packs (12) are less than half the number of packs (2) in each subgroup (13),
- establishing that a subgroup fulfils the predetermined second level acceptability criterion exclusively by using the second level sensors (5) associated with the sample packages (12), or the products contained in the sample packages (12), present in the respective subgroup (13), and
wherein the sample packages (12) of each subgroup (13) are placed in an outer peripheral area of the subgroup, wherein each subgroup has packages which are placed side by side and superimposed on each other to form an ideal cube or an ideal parallelogram, and in which each of the sample packages (12) is placed at a respective edge or vertex of the ideal cube or parallelogram; optionally the sample packages (12) are two or more in number for each subgroup (13).

16. The process of any one of claims 11-15, wherein the process comprises the further step of certifying that one or more subgroups (13) present in the load volume consist exclusively of products complying with a predetermined quality standard if, in a reference time interval during which the first level procedure is performed a plurality of times, the following occurs:
- the values of the first parameter comply with the predetermined first level acceptability criterion each time the first level procedure is performed, and
wherein process comprises the further step of certifying that one or more subgroups (13) present in the load volume consist exclusively of products complying with a predetermined quality standard if, in a reference time interval during which the first level procedure is performed a plurality of times, the following occurs:
- the values of the first parameter do not comply with the predetermined first level acceptability criterion during at least one execution of the first level procedure, and
- when the second level procedure is performed, it is verified that said one or more subgroups (13) comply with the predetermined second level acceptability criterion.

17. The process of any one of claims 11-16, wherein the process is executed by the infrastructure according to any one of claims 1 to 10.

18. A software product comprising instructions which when executed by a programmable control unit configure said programmable control unit to execute a process according to any one of claims 11 to 17,
optionally wherein said instructions are stored in a memory comprising an optical or magnetic disk memory or an EPROM-type memory or a volatile memory;
or wherein said instructions are carried by an electromagnetic signal, more optionally in a data package transmitted via the Internet.

## Patentansprüche

1. Infrastruktur zur Überwachung einer Ladung verderblicher Produkte, wobei die Ladung eine Vielzahl von Produkten umfasst, welche in jeweiligen Verpackungen (2) untergebracht sind, und in einem Ladevolumen (3) einer Lagereinheit oder eines Förderers angeordnet ist,
wobei:
- das Ladevolumen (3) mit einer vorbestimmten Anzahl von Sensoren erster Ebene (4) bereitgestellt ist, welche dazu eingerichtet sind, einen ersten Parameter zu detektieren, welcher sich auf eine in dem Ladevolumen selbst vorhandene Umgebung bezieht,
- das Ladevolumen (3) eine Mehrzahl von Untergruppen (13) der Vielzahl von Produkten umfasst, welche in dem Ladevolumen selbst vorhanden sind, wobei jede Untergruppe ihrerseits eine Mehrzahl von Produkten umfasst, welche zahlenmäßig einen Bruchteil und insbesondere einen ganzzahligen Teiler der Vielzahl von Produkten darstellen, welche in dem Ladevolumen vorhanden sind,
- jede der Verpackungen (2) oder der in den Verpackungen untergebrachten Produkte mit wenigstens einem Sensor zweiter Ebene (5) assoziiert ist, wobei jeder Sensor zweiter Ebene (5) wenigstens gegenüber einer Veränderung eines zweiten Parameters empfindlich ist, welcher sich auf eine Umgebung bezieht, welche an jede Verpackung angrenzt oder sich im Inneren jeder Verpackung befindet;
wobei die Infrastruktur (1) wenigstens einen Steuerungsüberwacher (6) umfasst, welcher dazu eingerichtet ist, einen Ablauf erster Ebene auszuführen, welcher die folgenden Schritte umfasst:
- Empfangen oder Bestimmen, auf Grundlage von Signalen, welche von den Sensoren erster Ebene emittiert werden, von Werten, welche der erste Parameter im Zeitverlauf annimmt, auf Grundlage von Detektionen des ersten Parameters, welche von der vorbestimmten Anzahl von Sensoren erster Ebene (4) durchgeführt werden,
- Feststellen, ob die Werte des ersten Parameters ein vorbestimmtes Zulässigkeitskriterium erster Ebene erfüllen oder nicht erfüllen,
- falls die Werte des ersten Parameters das vorbestimmte Zulässigkeitskriterium erster Ebene erfüllen, Wiederholen der Ausführung des Ablaufs erster Ebene,
wobei die Infrastruktur ferner dazu eingerichtet ist, einen Ablauf zweiter Ebene auszuführen, falls die Werte des ersten Parameters das vorbestimmte Zulässigkeitskriterium erster Ebene nicht erfüllen, wobei der Ablauf zweiter Ebene die folgenden Schritte umfasst:
- Feststellen, welche Untergruppen (13) ein vorbestimmtes Zulässigkeitskriterium zweiter Ebene erfüllen, unter Verwendung von Anzeigen bezüglich der Veränderung des zweiten Parameters, welche von wenigstens einem Teil der Sensoren zweiter Ebene (5) detektiert wird, welche mit den Produkten oder mit den Verpackungen (2) assoziiert sind, welche in jeder jeweiligen Untergruppe vorhanden sind.

2. Überwachungsinfrastruktur nach Anspruch 1, wobei die Sensoren zweiter Ebene (5) von einem anderen Typ sind als die Sensoren erster Ebene (4), wobei der Steuerungsüberwacher dazu eingerichtet ist, den Ablauf zweiter Ebene auszuführen, und
wobei der Steuerungsüberwacher (6) während einer Ausführung des Ablaufs zweiter Ebene dazu eingerichtet ist:
- kommunikativ mit den Sensoren erster Ebene (4) oder mit einer zwischengeschalteten Übertragungseinheit (7) verbunden zu sein, welche kommunikativ zwischen dem Steuerungsüberwacher (6) und den Sensoren erster Ebene (4) zwischengeschaltet ist, und
- die von den Sensoren erster Ebene (4) emittierten Signale oder direkt die Werte zu empfangen, welche der erste Parameter im Zeitverlauf annimmt;
und dazu eingerichtet ist:
- kommunikativ mit den Sensoren zweiter Ebene (5) oder mit einem Detektor (8) verbunden zu sein, welcher dazu eingerichtet ist, die von den Sensoren zweiter Ebene (5) emittierten Anzeigen auszulesen, und welcher kommunikativ zwischen dem Steuerungsüberwacher (6) und den Sensoren zweiter Ebene (5) zwischengeschaltet ist, und
- von dem Detektor (8) oder direkt von den Sensoren zweiter Ebene (5) die Anzeigen bezüglich der Veränderung des zweiten Parameters zu empfangen;
insbesondere wobei der Steuerungsüberwacher (6) in dem Ladevolumen (3) oder in einer entfernten Position in Bezug auf das Ladevolumen untergebracht ist.

3. Überwachungsinfrastruktur nach Anspruch 1 oder 2, wobei der Steuerungsüberwacher (6) dazu eingerichtet ist, den weiteren Schritt eines Zertifizierens auszuführen, dass die Produkte in dem Ladevolumen (3) alle einem vorbestimmten Qualitätsstandard entsprechen, falls in einem Referenzzeitintervall, während dessen der Ablauf erster Ebene mehrfach ausgeführt wird, folgendes verifiziert wird:
- die Werte des ersten Parameters erfüllen das vorbestimmte Zulässigkeitskriterium erster Ebene während jeder Ausführung des Ablaufs erster Ebene, oder
- der Ablauf zweiter Ebene wird niemals ausgeführt, oder
- falls der Ablauf zweiter Ebene ausgeführt wird, wird verifiziert, dass alle Untergruppen das vorbestimmte Zulässigkeitskriterium zweiter Ebene erfüllen;
optional wobei das Referenzzeitintervall gleich der Dauer eines Transports einer Ladung von einem Lieferpunkt zu einem Ziel ist.

4. Überwachungsinfrastruktur nach einem der vorhergehenden Ansprüche, welche die Sensoren erster Ebene umfasst, wobei jeder der Sensoren erster Ebene (4) eine jeweilige Steuereinheit (9), einen Speicher (10) und einen Sender (11) aufweist, welche direkt oder indirekt kommunikativ miteinander verbunden sind, und eingerichtet ist zum:
- Speichern eines oder mehrerer Werte, welche der erste Parameter im Zeitverlauf annimmt, und
- Übertragen eines oder mehrerer solcher Werte an den Steuerungsüberwacher, welcher dazu eingerichtet ist, den Ablauf erster Ebene auszuführen;
optional wobei jeder der Sensoren erster Ebene (4) einen Bluetooth-Sender, einen WLAN-Sender, ein RFID-Transponder-Tag, ein NFC aufweist, und wobei der erste Parameter an der Position in dem Ladevolumen detektiert wird, an welcher jeder Sensor erster Ebene (4) arbeitet, und einer der folgenden ist:
- die Temperatur, oder
- ein Parameter, welcher eine Funktion der Temperatur ist, oder
- die Konzentration eines vorbestimmten Gases oder Gasgemisches, oder
- die Konzentrationsvariation eines vorbestimmten Gases oder Gasgemisches,
wobei das vorbestimmte Zulässigkeitskriterium erster Ebene jeweils definiert ist durch:
- ein Temperaturintervall, oder
- ein Intervall von Werten des Parameters, welcher eine Funktion der Temperatur ist, oder
- ein Konzentrationsintervall eines vorbestimmten Gases oder Gasgemisches, oder
- ein Intervall der Konzentrationsvariation eines vorbestimmten Gases oder Gasgemisches, und
wobei eine Mehrzahl von Sensoren erster Ebene (4) in dem Ladevolumen (3) verteilt ist, optional gleichmäßig verteilt ist, wobei jeder der Sensoren erster Ebene (4) mit einem eindeutigen Identifikationscode assoziiert ist;
wobei die Sensoren erster Ebene (4) dazu eingerichtet sind, an den Steuerungsüberwacher (6) die Werte zu übertragen, welche der erste Parameter im Zeitverlauf annimmt, unter Assoziierung des jeweiligen eindeutigen Identifikationscodes jedes der ersten Sensoren, und
wobei der Steuerungsüberwacher (6) dazu eingerichtet ist, die Werte zu identifizieren, welche der erste Parameter im Zeitverlauf annimmt und welche von jedem der ersten Sensoren detektiert werden.

5. Überwachungsinfrastruktur nach einem der vorhergehenden Ansprüche, wobei der zweite Parameter einer der folgenden ist:
- die Temperatur einer Umgebung im Inneren der Verpackung (2), oder
- ein Parameter, welcher eine Funktion der Temperatur einer Umgebung im Inneren der Verpackung (2) ist, oder
- die Temperatur einer Außenfläche der Verpackung (2),
- ein Parameter, welcher eine Funktion der Temperatur einer Außenfläche der Verpackung (2) ist,
- die Konzentration eines vorbestimmten Gases oder einer vorbestimmten Substanz im Inneren der Verpackung (2),
- die Konzentrationsvariation eines vorbestimmten Gases oder einer vorbestimmten Substanz im Inneren der Verpackung, oder
- die Konzentration eines vorbestimmten Gases oder einer vorbestimmten Substanz an einer Außenfläche der Verpackung (2),
- die Konzentrationsvariation eines vorbestimmten Gases oder einer vorbestimmten Substanz an einer Außenfläche der Verpackung, oder
- der Säuregrad im Inneren der Verpackung (2) oder
- die Variation des Säuregrades im Inneren der Verpackung (2), und
wobei jeder der Sensoren zweiter Ebene (5), welcher mit einer jeweiligen Verpackung assoziiert ist, wenigstens eines der folgenden umfasst:
- eine Komponente, welche gegenüber der Temperatur oder einem zur Temperatur proportionalen Parameter empfindlich ist und von der Verpackung des Produkts getragen wird,
- eine Komponente, welche gegenüber der Temperatur oder einem zur Temperatur proportionalen Parameter empfindlich ist und im Inneren der Verpackung eingesetzt ist,
- eine Komponente, welche gegenüber der Konzentration oder der Konzentrationsvariation eines vorbestimmten Gases empfindlich ist und im Inneren der Verpackung eingesetzt ist,
- eine Komponente, welche gegenüber der Konzentration einer vorbestimmten Substanz oder der Variation einer vorbestimmten Substanz empfindlich ist und im Inneren der Verpackung eingesetzt ist,
- eine Komponente, welche gegenüber der Konzentration oder der Konzentrationsvariation eines vorbestimmten Gases empfindlich ist und von der Verpackung getragen wird,
- eine Komponente, welche gegenüber der Konzentration einer vorbestimmten Substanz oder der Variation einer vorbestimmten Substanz empfindlich ist und von der Verpackung getragen wird,
- eine Komponente, welche gegenüber dem pH-Wert oder der pH-Variation der Umgebung im Inneren der Verpackung empfindlich ist;
optional wobei jeder der Sensoren zweiter Ebene (5), welcher mit einer jeweiligen Verpackung (2) assoziiert ist,
- eine Einwegkomponente ist, welche auf die Verpackung (2) aufgebracht oder in die Verpackung eingesetzt ist, oder
- eine Komponente ist, welche direkt auf die Verpackung (2) aufgedruckt ist, optional auf eine Kunststofffolie oder auf eine Kunststoff- oder Papierschale, welche Teil der Verpackung (2) ist.

6. Überwachungsinfrastruktur nach einem der vorhergehenden Ansprüche, wobei jeder der Sensoren zweiter Ebene (5) dazu eingerichtet ist, wenigstens eines der folgenden bereitzustellen:
- eine optional visuelle Anzeige vom Ja/Nein-Typ darüber, ob das Produkt im Inneren der jeweiligen Verpackung eine bestimmte Temperatur für ein bestimmtes Zeitintervall überschritten hat oder nicht überschritten hat;
- eine optional visuelle Anzeige, welche proportional zur maximalen Temperatur ist, welche das Produkt im Inneren der jeweiligen Verpackung erreicht hat;
- eine optional visuelle Anzeige vom Ja/Nein-Typ darüber, ob die Zone der Verpackung, in welcher der Sensor zweiter Ebene vorhanden ist, eine bestimmte Temperatur für ein bestimmtes Zeitintervall überschritten hat oder nicht überschritten hat;
- eine optional visuelle Anzeige, welche proportional zur maximalen Temperatur ist, welche in der Zone der Verpackung erreicht wurde, in welcher der Sensor zweiter Ebene vorhanden ist;
- eine optional visuelle Anzeige vom Ja/Nein-Typ darüber, ob im Inneren der jeweiligen Verpackung eine bestimmte Konzentration oder Konzentrationsvariation eines vorbestimmten Gases oder Gasgemisches für ein bestimmtes Zeitintervall überschritten wurde oder nicht überschritten wurde;
- eine optional visuelle Anzeige, welche proportional zur maximalen Konzentration eines vorbestimmten Gases oder Gasgemisches ist, welche im Inneren der jeweiligen Verpackung erreicht wurde;
- eine optional visuelle Anzeige vom Ja/Nein-Typ darüber, ob im Inneren der jeweiligen Verpackung eine bestimmte Konzentration oder Konzentrationsvariation einer vorbestimmten Substanz oder Gruppe von Substanzen für ein bestimmtes Zeitintervall überschritten wurde oder nicht überschritten wurde;
- eine optional visuelle Anzeige, welche proportional zur maximalen Konzentration einer vorbestimmten Substanz oder Gruppe von Substanzen im Inneren der jeweiligen Verpackung ist;
- eine optional visuelle Anzeige vom Ja/Nein-Typ darüber, ob an einer Oberfläche der jeweiligen Verpackung eine bestimmte Konzentration oder Konzentrationsvariation eines vorbestimmten Gases oder Gasgemisches für ein bestimmtes Zeitintervall überschritten wurde oder nicht überschritten wurde;
- eine optional visuelle Anzeige, welche proportional zur maximalen Konzentration eines vorbestimmten Gases oder Gasgemisches ist, welche an einer Oberfläche der jeweiligen Verpackung erreicht wurde;
- eine optional visuelle Anzeige vom Ja/Nein-Typ darüber, ob im Inneren der jeweiligen Verpackung ein bestimmter pH-Wert oder eine bestimmte pH-Variation für ein bestimmtes Zeitintervall überschritten wurde oder nicht überschritten wurde;
- eine optional visuelle Anzeige, welche proportional zum maximalen oder minimalen pH-Wert ist, welcher im Inneren der jeweiligen Verpackung erreicht wurde;
wobei das vorbestimmte Zulässigkeitskriterium zweiter Ebene für eine bestimmte Untergruppe nur dann als erfüllt gilt, wenn die optional visuelle Anzeige, welche von jedem der Sensoren zweiter Ebene (5) bereitgestellt wird, welche in dem Ablauf zweiter Ebene für diese Untergruppe verwendet werden, einen jeweiligen Verifizierungstest erfüllt.

7. Überwachungsinfrastruktur nach einem der vorhergehenden Ansprüche, wobei der Schritt des Ablaufs zweiter Ebene des Feststellens, welche Untergruppen ein vorbestimmtes Zulässigkeitskriterium zweiter Ebene erfüllen, umfasst:
- Empfangen einer Identifikation, für jede Untergruppe (13), einer vorbestimmten Anzahl von Stichprobenverpackungen (12), wobei die Stichprobenverpackungen (12) zahlenmäßig geringer sind als die Gesamtanzahl von Verpackungen in jeder Untergruppe (13), insbesondere wobei die Stichprobenverpackungen zahlenmäßig geringer sind als die Hälfte der Verpackungen (2), welche in jeder Untergruppe (13) vorhanden sind,
- Feststellen, dass eine Untergruppe das vorbestimmte Zulässigkeitskriterium zweiter Ebene erfüllt, ausschließlich unter Verwendung der Sensoren zweiter Ebene (5), welche mit den Stichprobenverpackungen (12) oder mit den in den Stichprobenverpackungen (12) enthaltenen Produkten assoziiert sind, welche in der jeweiligen Untergruppe (13) vorhanden sind;
wobei die Stichprobenverpackungen (12) jeder Untergruppe (13) in einer äußeren Randzone der Untergruppe selbst angeordnet sind;
optional wobei, wenn jede Untergruppe (13) Verpackungen (2) aufweist, welche aneinander angrenzen und übereinander gestapelt sind, um einen Würfel oder ein ideales Parallelogramm zu bilden, jede der Stichprobenverpackungen (12) an einer jeweiligen Kante oder einer jeweiligen Ecke des Würfels oder idealen Parallelogramms angeordnet ist,
weiter optional wobei die Stichprobenverpackungen (12) zwei oder mehr, noch weiter optional 3 bis 6, für jede Untergruppe (13) sind, und
wobei jede Verpackung (2), insbesondere jeder der Sensoren zweiter Ebene (5) jeder Verpackung (2), mit einem eindeutigen Identifikationscode assoziiert ist, und wobei der Schritt des Feststellens, welche Untergruppen ein vorbestimmtes Zulässigkeitskriterium zweiter Ebene erfüllen, für jede Untergruppe umfasst:
- direktes Empfangen, oder von dem Detektor (8), wenigstens einer Anzeige bezüglich des zweiten Parameters, welche von jedem der abgefragten oder beobachteten zweiten Sensoren (5) emittiert oder angezeigt wird,
- Empfangen, in Assoziation mit jeder Anzeige, des jeweiligen eindeutigen Identifikationscodes der jeweiligen Verpackung (2) jedes der abgefragten oder beobachteten zweiten Sensoren (5).

8. Überwachungsinfrastruktur nach Anspruch 7, wobei der Steuerungsüberwacher (6) oder der Detektor (8) dazu eingerichtet ist, in jeder Untergruppe (13) die Stichprobenverpackungen (12) zu identifizieren, indem ein Abfrageprotokoll verwendet wird, welches eine Liste von eindeutigen Identifikationscodes der Verpackungen (2) umfasst, und wobei der Steuerungsüberwacher (6) oder der Detektor (8) dazu eingerichtet ist, als Stichprobenverpackungen (12) diejenigen auszuwählen, welche zu der in dem Abfrageprotokoll vorhandenen Liste gehören, und
wobei der Ablauf zweiter Ebene einen Verifizierungsschritt umfasst, welcher direkt von dem Steuerungsüberwacher (6) oder von dem Detektor (8) durchgeführt wird, in welchem die in jeder Untergruppe (13) identifizierten Stichprobenverpackungen (12) alle und ausschließlich diejenigen sind, welche in der oder in einer Liste des eines Abfrageprotokolls vorhanden sind, welches die eindeutigen Identifikationscodes der auszuwählenden Stichprobenverpackungen (12) umfasst, optional wobei das Abfrageprotokoll in dem Steuerungsüberwacher (6) gespeichert ist.

9. Überwachungsinfrastruktur nach einem der Ansprüche 2 bis 8, wobei die Anzeige, welche von jedem der Sensoren zweiter Ebene (5) bereitgestellt wird, von dem Detektor (8) ausgelesen wird, welcher kommunikativ mit dem Steuerungsüberwacher (6) verbunden ist, und an diesen übertragen wird, insbesondere wobei der Detektor umfasst:
- wenigstens einen optischen Scanner oder eine Videokamera, falls die Anzeige visueller Art ist, oder
- wenigstens einen Funkwellendetektor, falls die Anzeige elektromagnetischer Art ist, oder
- wenigstens einen akustischen Detektor, falls die Anzeige akustischer Art ist.

10. Überwachungsinfrastruktur nach einem der vorhergehenden Ansprüche, wobei der Steuerungsüberwacher einen oder mehrere Digitalcomputer und/oder einen oder mehrere Analogcomputer umfasst und wobei der Steuerungsüberwacher (6) dazu eingerichtet ist, periodisch oder in regelmäßigen Intervallen oder infolge eines Befehls oder infolge der Detektion eines Ereignisses an ein verteiltes Register (16) vom Blockchain-Typ, welches mit dem Steuerungsüberwacher (6) verbunden ist, wenigstens eines oder mehrere, optional alle, der folgenden zu kommunizieren:
- den eindeutigen Identifikationscode bezüglich jedes Sensors erster Ebene (4),
- das Wertepaar, welches aus einem Wert des ersten Parameters und einem Abtastzeitpunkt gebildet wird, welches von jedem Sensor erster Ebene (4) während einer Ausführung des Ablaufs erster Ebene detektiert wird,
- den eindeutigen Identifikationscode jedes Sensors zweiter Ebene (5),
- das Wertepaar, welches aus einer Anzeige bezüglich des zweiten Parameters und einem Detektionszeitpunkt gebildet wird, welches von dem Detektor (8) für jeden abgefragten Sensor zweiter Ebene aufgezeichnet wird, und
wobei der Steuerungsüberwacher (6) dazu eingerichtet ist, zu zertifizieren, welche eine oder mehrere Untergruppen (13), welche in dem Ladevolumen (3) vorhanden sind, ausschließlich aus Produkten bestehen, welche einem vorbestimmten Qualitätsstandard entsprechen, falls in einem Referenzzeitintervall, während dessen der Ablauf erster Ebene mehrfach ausgeführt wird, wie folgt verifiziert wird:
- die Werte des ersten Parameters erfüllen das vorbestimmte Zulässigkeitskriterium erster Ebene bei jeder Ausführung des Ablaufs erster Ebene,
oder falls
- die Werte des ersten Parameters das vorbestimmte Zulässigkeitskriterium erster Ebene während wenigstens einer Ausführung des Ablaufs erster Ebene nicht erfüllen, und
- sobald der Ablauf zweiter Ebene ausgeführt wird, verifiziert wird, dass die eine oder die mehreren Untergruppen (13) das vorbestimmte Zulässigkeitskriterium zweiter Ebene erfüllen;
optional wobei das Referenzzeitintervall gleich der Dauer eines Transports der Ladung von einem Lieferpunkt zu einem Ziel ist.

11. Computerimplementiertes Verfahren zur Überwachung einer Ladung verderblicher Produkte, wobei die Ladung eine Vielzahl von Produkten umfasst, welche in jeweiligen Verpackungen (2) untergebracht sind, und in einem Ladevolumen einer Lagereinheit oder eines Förderers angeordnet ist, wobei:
- das Ladevolumen (3) mit einer vorbestimmten Anzahl von Sensoren erster Ebene (4) bereitgestellt ist, welche dazu eingerichtet sind, einen ersten Parameter zu detektieren, welcher sich auf eine in dem Ladevolumen (3) selbst vorhandene Umgebung bezieht,
- das Ladevolumen (3) eine Mehrzahl von Untergruppen (13) der Vielzahl von Produkten umfasst, welche in diesem Ladevolumen vorhanden sind, wobei jede Untergruppe (13) eine Mehrzahl von Produkten umfasst, welche zahlenmäßig einen Bruchteil und insbesondere einen ganzzahligen Teiler der Vielzahl von Produkten darstellen, welche in dem Ladevolumen (3) vorhanden sind,
- jede der Verpackungen (2) oder der in den Verpackungen (2) untergebrachten Produkte mit wenigstens einem Sensor zweiter Ebene (5) assoziiert ist, wobei jeder Sensor zweiter Ebene (5) gegenüber wenigstens einer Veränderung eines zweiten Parameters empfindlich ist, welcher sich auf jede Verpackung (2) oder auf eine Umgebung im Inneren jeder Verpackung (2) bezieht;
wobei das Verfahren die Ausführung eines Ablaufs erster Ebene umfasst, welcher die folgenden Schritte umfasst:
- Bestimmen von Werten, welche der erste Parameter im Zeitverlauf annimmt, auf Grundlage von Detektionen des ersten Parameters, welche von der vorbestimmten Anzahl von Sensoren erster Ebene (4) durchgeführt werden,
- Feststellen, ob die Werte des ersten Parameters ein vorbestimmtes Zulässigkeitskriterium erster Ebene erfüllen oder nicht erfüllen,
- falls die Werte des ersten Parameters das vorbestimmte Zulässigkeitskriterium erster Ebene erfüllen, Wiederholen der Ausführung des Ablaufs erster Ebene;
und wobei das Verfahren einen Ablauf zweiter Ebene umfasst, welcher durchgeführt wird, falls die Werte des ersten Parameters das vorbestimmte Zulässigkeitskriterium erster Ebene nicht erfüllen, wobei der Ablauf zweiter Ebene die folgenden Schritte umfasst:
- Feststellen, welche Untergruppen (13) ein vorbestimmtes Zulässigkeitskriterium zweiter Ebene erfüllen, unter Verwendung von Anzeigen bezüglich der Veränderung des zweiten Parameters, welche von wenigstens einem Teil der Sensoren zweiter Ebene (5) detektiert wird, welche mit den Produkten oder mit den Verpackungen (2) assoziiert sind, welche in jeder jeweiligen Untergruppe vorhanden sind.

12. Verfahren nach Anspruch 11, wobei die Sensoren zweiter Ebene (5) von einem anderen Typ sind als die Sensoren erster Ebene (4), und/oder wobei das Ladevolumen (3) eine Mehrzahl von Untergruppen (13) von Produkten umfasst, wobei jede Untergruppe (13) identifizierbar und von den anderen Untergruppen unterscheidbar ist, insbesondere wobei jede Untergruppe in einem jeweiligen Gehäuse untergebracht oder auf einer jeweiligen Palette angeordnet ist, und/oder
wobei das Verfahren den weiteren Schritt eines Zertifizierens umfasst, dass alle Produkte in dem Ladevolumen (3) einem vorbestimmten Qualitätsstandard entsprechen, falls in einem Referenzzeitintervall, während dessen der Ablauf erster Ebene mehrfach durchgeführt wird, folgendes verifiziert wird:
- die Werte des ersten Parameters erfüllen das vorbestimmte Zulässigkeitskriterium erster Ebene während jeder Ausführung des Ablaufs erster Ebene;
optional wobei das Referenzzeitintervall gleich der Dauer eines Transports einer Ladung von einem Lieferpunkt zu einem Ziel ist.

13. Verfahren nach einem der vorhergehenden zwei Ansprüche, wobei das Verfahren den weiteren Schritt eines Zertifizierens umfasst, dass die Produkte in dem Ladevolumen (3) alle einem vorbestimmten Qualitätsstandard entsprechen, falls in einem Referenzzeitintervall, während dessen der Ablauf erster Ebene mehrfach durchgeführt wird, folgendes verifiziert wird:
- der Ablauf zweiter Ebene wird niemals durchgeführt;
und wobei das Verfahren den weiteren Schritt eines Zertifizierens umfasst, dass alle Produkte in dem Ladevolumen (3) einem vorbestimmten Qualitätsstandard entsprechen, falls in einem Referenzzeitintervall, während dessen der Ablauf erster Ebene mehrfach durchgeführt wird, folgendes eintritt:
- falls der Ablauf zweiter Ebene durchgeführt wird, wird verifiziert, dass alle Untergruppen das vorbestimmte Zulässigkeitskriterium zweiter Ebene erfüllen, und
wobei der Ablauf zweiter Ebene den zusätzlichen Schritt eines Meldens von Untergruppen umfasst, welche das vorbestimmte Zulässigkeitskriterium zweiter Ebene nicht erfüllen.

14. Verfahren nach einem der Ansprüche 11-13, wobei der erste Parameter an der Position im Inneren des Ladevolumens detektiert wird, an welcher jeder Sensor erster Ebene (4) arbeitet; insbesondere wobei eine Mehrzahl von Sensoren erster Ebene (4) in dem Ladevolumen verteilt ist, optional gleichmäßig verteilt ist, und
wobei das vorbestimmte Zulässigkeitskriterium erster Ebene durch einen Temperaturbereich oder durch einen Bereich von Parameterzulässigkeitswerten als Funktion der Temperatur oder der Temperaturvariation definiert ist;
oder wobei das vorbestimmte Zulässigkeitskriterium erster Ebene durch einen Konzentrationsbereich oder eine Konzentrationsvariation eines vorbestimmten Gases oder Gasgemisches definiert ist, und
wobei der zweite Parameter die Temperatur oder ein temperaturabhängiger Parameter jeder Verpackung (2) oder einer Umgebung im Inneren jeder Verpackung ist; oder der zweite Parameter die Temperatur einer Außenfläche der Verpackung (2) ist; oder der zweite Parameter die Temperatur im Inneren jeder Verpackung (2) ist; oder der zweite Parameter die Konzentration oder Konzentrationsvariation eines vorbestimmten Gases im Inneren der Verpackung (2) ist; oder der zweite Parameter die Konzentration oder Konzentrationsvariation einer vorbestimmten Substanz im Inneren der Verpackung ist; oder der zweite Parameter der Säuregrad im Inneren der Verpackung (2) oder die Variation des Säuregrades in jeder Verpackung (2) ist.

15. Verfahren nach einem der Ansprüche 11-14, wobei jeder der Sensoren zweiter Ebene (5) vorsieht, dass das vorbestimmte Zulässigkeitskriterium zweiter Ebene für eine gegebene Untergruppe (13) nur dann als erfüllt gilt, wenn die optional visuelle Anzeige, welche von jedem der Sensoren zweiter Ebene (5) bereitgestellt wird, welche in dem Ablauf zweiter Ebene für diese Untergruppe verwendet werden, einen jeweiligen Verifizierungstest erfüllt, und
wobei der Schritt des Ablaufs zweiter Ebene des Bestimmens, welche Untergruppen ein vorbestimmtes Zulässigkeitskriterium zweiter Ebene erfüllen, umfasst:
- Identifizieren in jeder Untergruppe (13) einer vorbestimmten Anzahl von Stichprobenpaketen (12), wobei die Stichprobenpakete (12) zahlenmäßig geringer sind als die Gesamtanzahl von Paketen (2) in jeder Untergruppe (13), insbesondere wobei die Stichprobenpakete (12) zahlenmäßig geringer sind als die Hälfte der Anzahl von Paketen (2) in jeder Untergruppe (13),
- Feststellen, dass eine Untergruppe das vorbestimmte Zulässigkeitskriterium zweiter Ebene erfüllt, ausschließlich unter Verwendung der Sensoren zweiter Ebene (5), welche mit den Stichprobenverpackungen (12) oder den in den Stichprobenverpackungen (12) enthaltenen Produkten assoziiert sind, welche in der jeweiligen Untergruppe (13) vorhanden sind, und
wobei die Stichprobenverpackungen (12) jeder Untergruppe (13) in einem äußeren Randbereich der Untergruppe angeordnet sind, wobei jede Untergruppe Verpackungen aufweist, welche nebeneinander angeordnet und übereinander gestapelt sind, um einen idealen Würfel oder ein ideales Parallelogramm zu bilden, und in welcher jede der Stichprobenverpackungen (12) an einer jeweiligen Kante oder Ecke des idealen Würfels oder Parallelogramms angeordnet ist; optional wobei die Stichprobenverpackungen (12) für jede Untergruppe (13) zwei oder mehr sind.

16. Verfahren nach einem der Ansprüche 11-15, wobei das Verfahren den weiteren Schritt eines Zertifizierens umfasst, dass eine oder mehrere Untergruppen (13), welche in dem Ladevolumen vorhanden sind, ausschließlich aus Produkten bestehen, welche einem vorbestimmten Qualitätsstandard entsprechen, falls in einem Referenzzeitintervall, während dessen der Ablauf erster Ebene mehrfach durchgeführt wird, folgendes eintritt:
- die Werte des ersten Parameters erfüllen das vorbestimmte Zulässigkeitskriterium erster Ebene jedes Mal, wenn der Ablauf erster Ebene durchgeführt wird, und
wobei das Verfahren den weiteren Schritt eines Zertifizierens umfasst, dass eine oder mehrere Untergruppen (13), welche in dem Ladevolumen vorhanden sind, ausschließlich aus Produkten bestehen, welche einem vorbestimmten Qualitätsstandard entsprechen, falls in einem Referenzzeitintervall, während dessen der Ablauf erster Ebene mehrfach durchgeführt wird, folgendes eintritt:
- die Werte des ersten Parameters erfüllen das vorbestimmte Zulässigkeitskriterium erster Ebene während wenigstens einer Ausführung des Ablaufs erster Ebene nicht, und
- wenn der Ablauf zweiter Ebene durchgeführt wird, wird verifiziert, dass die eine oder die mehreren Untergruppen (13) das vorbestimmte Zulässigkeitskriterium zweiter Ebene erfüllen.

17. Verfahren nach einem der Ansprüche 11-16, wobei das Verfahren von der Infrastruktur nach einem der Ansprüche 1 bis 10 ausgeführt wird.

18. Softwareprodukt, welches Anweisungen umfasst, welche, wenn sie von einer programmierbaren Steuereinheit ausgeführt werden, die programmierbare Steuereinheit dazu einrichten, ein Verfahren nach einem der Ansprüche 11 bis 17 auszuführen,
optional wobei die Anweisungen in einem Speicher gespeichert sind, welcher einen optischen oder magnetischen Datenträgerspeicher oder einen Speicher vom EPROM-Typ oder einen flüchtigen Speicher umfasst;
oder wobei die Anweisungen von einem elektromagnetischen Signal getragen werden, weiter optional in einem Datenpaket, welches über das Internet übertragen wird.

## Revendications

1. Infrastructure de surveillance d'un chargement de produits périssables, ledit chargement comprenant une multiplicité de produits logés dans des emballages (2) respectifs et placés dans un volume de chargement (3) d'une unité de stockage ou d'un convoyeur,
dans laquelle :
- le volume de chargement (3) est fourni avec un nombre prédéterminé de capteurs de premier niveau (4) configurés pour détecter un premier paramètre relatif à un environnement présent dans le volume de chargement lui-même,
- le volume de chargement (3) comprend une pluralité de sous-groupes (13) de ladite multiplicité de produits présents dans le volume de chargement lui-même, dans laquelle chaque sous-groupe à son tour comprend une pluralité de produits qui sont, en nombre, une fraction et en particulier un sous-multiple de ladite multiplicité de produits présents dans le volume de chargement,
- chacun des emballages (2) ou des produits logés dans lesdits emballages est associé à au moins un capteur de second niveau (5), chaque capteur de second niveau (5) étant sensible au moins à une altération d'un second paramètre par rapport à un environnement adjacent à, ou à l'intérieur de, chaque emballage ;
ladite infrastructure (1) comprenant au moins un superviseur de contrôle (6) configuré pour exécuter une procédure de premier niveau qui comprend les étapes suivantes consistant à :
- recevoir, ou déterminer sur la base des signaux émis par les capteurs de premier niveau, des valeurs prises par ledit premier paramètre dans le temps sur la base de détections du premier paramètre exécutées par ledit nombre prédéterminé de capteurs de premier niveau (4),
- établir si lesdites valeurs du premier paramètre sont conformes, ou ne sont pas conformes, à un critère prédéterminé d'acceptabilité de premier niveau,
- si les valeurs du premier paramètre sont conformes au critère prédéterminé d'acceptabilité de premier niveau, répéter l'exécution de la procédure de premier niveau,
ladite infrastructure étant également configurée pour exécuter une procédure de second niveau si les valeurs du premier paramètre ne sont pas conformes au critère prédéterminé d'acceptabilité de premier niveau, la procédure de second niveau comprenant les étapes suivantes consistant à :
- établir quels sous-groupes (13) sont conformes à un critère prédéterminé d'acceptabilité de second niveau, en utilisant des indications par rapport à l'altération du second paramètre détectée par au moins une partie des capteurs de second niveau (5) associés aux produits ou aux emballages (2) présents dans chaque sous-groupe respectif.

2. Infrastructure de surveillance selon la revendication 1, dans laquelle les capteurs de second niveau (5) sont d'un type différent de celui des capteurs de premier niveau (4),
dans laquelle le superviseur de contrôle est configuré pour exécuter la procédure de second niveau, et
dans laquelle durant l'exécution de la procédure de second niveau le superviseur de contrôle (6) est configuré pour :
- être raccordé de manière à pouvoir communiquer aux capteurs de premier niveau (4) ou à une unité de transmission intermédiaire (7) interposée de manière à pouvoir communiquer entre le superviseur de contrôle (6) et les capteurs de premier niveau (4), et
- recevoir les signaux émis par les capteurs de premier niveau (4) ou directement les valeurs prises par le premier paramètre dans le temps ;
et pour :
- être raccordé de manière à pouvoir communiquer avec les capteurs de second niveau (5) ou avec un détecteur (8), configuré pour lire les indications émises par les capteurs de second niveau (5), interposé de manière à pouvoir communiquer entre le superviseur de contrôle (6) et les capteurs de second niveau (5) et
- recevoir, dudit détecteur (8) ou directement desdits capteurs de second niveau (5), les indications concernant l'altération du second paramètre ;
en particulier dans laquelle le superviseur de contrôle (6) est logé dans le volume de chargement (3) ou dans une position distante par rapport au volume de chargement.

3. Infrastructure de surveillance selon la revendication 1 ou 2, dans laquelle le superviseur de contrôle (6) est configuré pour exécuter l'étape supplémentaire de certification que les produits dans le volume de chargement (3) se conforment tous à un standard de qualité prédéterminé si, dans un intervalle de temps de référence durant lequel la procédure de premier niveau est exécutée une pluralité de fois, ce qui suit est vérifié :
- les valeurs du premier paramètre sont conformes au critère prédéterminé d'acceptabilité de premier niveau durant chaque exécution de la procédure de premier niveau, ou la procédure de second niveau n'est jamais exécutée, ou
- si la procédure de second niveau est exécutée, il est vérifié que tous les sous-groupes sont conformes au critère prédéterminé d'acceptabilité de second niveau ;
éventuellement dans laquelle l'intervalle de temps de référence est égal à la durée d'un transport d'un chargement d'un point de distribution vers une destination.

4. Infrastructure de surveillance selon l'une quelconque des revendications précédentes comprenant les capteurs de premier niveau, dans laquelle chacun des capteurs de premier niveau (4) comprend une unité de contrôle (9) respective, une mémoire (10) et un transmetteur (11), raccordés l'un à l'autre de manière à pouvoir communiquer d'une manière directe ou indirecte, et est configuré pour :
- stocker une ou plusieurs valeurs prises dans le temps par le premier paramètre et
- transmettre une telle ou de telles valeurs au superviseur de contrôle configuré pour exécuter la procédure de premier niveau ;
éventuellement dans laquelle chacun desdits capteurs de premier niveau (4) comprend un transmetteur Bluetooth, un transmetteur Wi-Fi, une étiquette transpondeur RFID, un NFC, et
dans laquelle le premier paramètre est détecté à la position dans le volume de chargement dans laquelle chaque capteur de premier niveau (4) fonctionne et est l'un de ce qui suit :
- la température, ou
- un paramètre qui est une fonction de la température, ou
- la concentration d'un gaz ou d'un mélange de gaz prédéterminé, ou
- la variation de concentration d'un gaz ou d'un mélange de gaz prédéterminé,
dans laquelle ledit critère prédéterminé d'acceptabilité de premier niveau est respectivement défini par
- un intervalle de température, ou
- un intervalle de valeurs du paramètre qui est une fonction de la température, ou
- un intervalle de concentration d'un gaz ou d'un mélange de gaz prédéterminé, ou
- un intervalle dans la variation de concentration d'un gaz ou d'un mélange de gaz prédéterminé, et
dans laquelle une pluralité de capteurs de premier niveau (4) sont distribués, éventuellement uniformément distribués, dans le volume de chargement (3), chacun des capteurs de premier niveau (4) étant associé à un code d'identification unique ;
dans laquelle les capteurs de premier niveau (4) sont configurés pour transmettre, au superviseur de contrôle (6), les valeurs prises dans le temps par le premier paramètre associant le code d'identification unique respectif de chacun des premiers capteurs, et
dans laquelle le superviseur de contrôle (6) est configuré pour identifier les valeurs prises dans le temps par le premier paramètre détectées par chacun desdits premiers capteurs.

5. Infrastructure de surveillance selon l'une quelconque des revendications précédentes, dans laquelle le second paramètre est l'un des suivants :
- la température d'un environnement à l'intérieur de l'emballage (2), ou
- un paramètre qui est une fonction de la température d'un environnement à l'intérieur de l'emballage (2), ou la température d'une surface externe de l'emballage (2),
- un paramètre qui est une fonction de la température d'une surface externe de l'emballage (2),
- la concentration d'un gaz prédéterminé ou d'une substance prédéterminée à l'intérieur de l'emballage (2),
- la variation de concentration d'un gaz ou d'une substance prédéterminé·e à l'intérieur de l'emballage, ou
- la concentration d'un gaz prédéterminé ou d'une substance prédéterminée sur une surface externe de l'emballage (2),
- la variation de concentration d'un gaz ou d'une substance prédéterminé·e sur une surface externe de l'emballage, ou
- le degré d'acidité présent à l'intérieur de l'emballage (2) ou
- la variation du degré d'acidité à l'intérieur de l'emballage (2), et
dans laquelle chacun des capteurs de second niveau (5) associés à un emballage respectif comprend au moins l'un de ce qui suit :
- un composant sensible à la température ou à un paramètre proportionnel à la température, porté par l'emballage du produit,
- un composant sensible à la température ou à un paramètre proportionnel à la température inséré à l'intérieur de l'emballage,
- un composant, sensible à la concentration ou à la variation de concentration d'un gaz prédéterminé, inséré à l'intérieur de l'emballage,
- un composant, sensible à la concentration d'une substance prédéterminée ou à la variation d'une substance prédéterminée, inséré à l'intérieur de l'emballage,
- un composant, sensible à la concentration ou à la variation de concentration d'un gaz prédéterminé, porté par l'emballage,
- un composant, sensible à la concentration d'une substance prédéterminée ou à la variation d'une substance prédéterminée, porté par l'emballage,
- un composant sensible au pH ou à la variation de pH de l'environnement à l'intérieur de l'emballage ;
éventuellement dans laquelle chacun des capteurs de second niveau (5) associés à un emballage respectif (2) est
- un composant jetable appliqué à l'emballage (2) ou inséré dans l'emballage, ou
- un composant imprimé directement sur l'emballage (2), éventuellement sur un film en matière plastique ou sur un plateau en matière plastique ou en papier qui fait partie de l'emballage (2).

6. Infrastructure de surveillance selon l'une quelconque des revendications précédentes, dans laquelle chacun des capteurs de second niveau (5) est configuré pour fournir au moins l'un de ce qui suit :
- une indication, éventuellement visuelle, du type oui ou non du fait que le produit à l'intérieur de l'emballage respectif a excédé ou n'a pas excédé une certaine température sur un intervalle de temps spécifique ;
- une indication, éventuellement visuelle, proportionnelle à la température maximale atteinte par le produit à l'intérieur de l'emballage respectif ;
- une indication, éventuellement visuelle, du type oui ou non du fait que la zone de l'emballage où le capteur de second niveau est présent a excédé ou n'a pas excédé une certaine température sur un intervalle de temps spécifique ;
- une indication, éventuellement visuelle, proportionnelle à la température maximale atteinte dans la zone de l'emballage où le capteur de second niveau est présent ;
- une indication, éventuellement visuelle, du type oui ou non du fait qu'à l'intérieur de l'emballage respectif, une certaine concentration ou variation de concentration d'un gaz ou d'un mélange de gaz prédéterminé a été dépassée ou pas dépassée sur un intervalle de temps spécifique ;
- une indication, éventuellement visuelle, proportionnelle à la concentration maximale d'un gaz ou d'un mélange de gaz prédéterminé atteinte à l'intérieur de l'emballage respectif ;
- une indication, éventuellement visuelle, du type oui ou non du fait qu'à l'intérieur de l'emballage respectif, une certaine concentration ou variation de concentration d'une substance ou d'un groupe de substances prédéterminée·s a été dépassée ou pas dépassée sur un intervalle de temps spécifique ;
- une indication, éventuellement visuelle, proportionnelle à la concentration maximale d'une substance ou d'un groupe de substances prédéterminée·s à l'intérieur de l'emballage respectif ;
- une indication, éventuellement visuelle, du type oui ou non du fait qu'à une surface de l'emballage respectif une certaine concentration ou variation de concentration d'un gaz ou d'un mélange de gaz prédéterminé a été dépassée ou pas dépassée sur un intervalle de temps spécifique ;
- une indication, éventuellement visuelle, proportionnelle à la concentration maximale d'un gaz ou d'un mélange de gaz prédéterminé atteinte à une surface de l'emballage respectif ;
- une indication, éventuellement visuelle, du type oui ou non du fait qu'à l'intérieur de l'emballage respectif un certain pH ou une certaine variation de pH a été dépassé·e ou pas dépassé·e sur un intervalle de temps spécifique ;
- une indication, éventuellement visuelle, proportionnelle au pH maximal ou minimal atteint à l'intérieur de l'emballage respectif ;
dans laquelle, le critère prédéterminé d'acceptabilité de second niveau est considéré comme satisfait pour un sous-groupe spécifique uniquement si l'indication, éventuellement visuelle, fournie par chacun des capteurs de second niveau (5) utilisés dans la procédure de second niveau pour un tel sous-groupe satisfait un test respectif de vérification.

7. Infrastructure de surveillance selon l'une quelconque des revendications précédentes, dans laquelle l'étape de la procédure de second niveau d'établissement des sous-groupes qui sont conformes à un critère prédéterminé d'acceptabilité de second niveau comprend les étapes consistant à :
- recevoir une identification, pour chaque sous-groupe (13), d'un nombre prédéterminé d'emballages d'échantillon (12), lesdits emballages d'échantillon (12) se trouvant dans un nombre inférieur au nombre total d'emballages dans chaque sous-groupe (13), en particulier dans laquelle lesdits emballages d'échantillon se trouvent dans un nombre inférieur à la moitié des emballages (2) présents dans chaque sous-groupe (13),
- établir qu'un sous-groupe est conforme au critère prédéterminé d'acceptabilité de second niveau en utilisant uniquement les capteurs de second niveau (5) associés aux emballages d'échantillon (12), ou aux produits contenus dans les emballages d'échantillon (12), présents dans le sous-groupe (13) respectif ;
dans laquelle les emballages d'échantillon (12) de chaque sous-groupe (13) sont placés dans une zone périphérique externe du sous-groupe lui-même ;
éventuellement dans laquelle lorsque chaque sous-groupe (13) présente des emballages (2) adjacents et surimposés l'un sur l'autre pour former un cube ou un parallélogramme idéal, chacun des emballages d'échantillon (12) est placé au niveau d'un bord respectif ou d'un sommet respectif du cube ou du parallélogramme idéal,
plus facultativement dans laquelle les emballages d'échantillon (12) sont de deux ou plusieurs, encore plus facultativement de 3 à 6, pour chaque sous-groupe (13), et
dans laquelle chaque emballage (2), en particulier chacun des capteurs de second niveau (5) de chaque emballage (2), est associé à un code d'identification unique, et dans laquelle l'étape d'établissement des sous-groupes qui sont conformes à un critère prédéterminé d'acceptabilité de second niveau comprend, pour chaque sous-groupe les étapes consistant à :
- recevoir directement, ou dudit détecteur (8), au moins une indication relative au second paramètre, émise ou affichée par chacun des seconds capteurs (5) interrogés ou observés,
- recevoir, en association à chaque indication, le code d'identification unique respectif de l'emballage respectif (2) de chacun des seconds capteurs (5) interrogés ou observés.

8. Infrastructure de surveillance selon la revendication 7, dans laquelle le superviseur de contrôle (6) ou le détecteur (8) est configuré pour identifier, dans chaque sous-groupe (13), les emballages d'échantillon (12) en utilisant un protocole d'interrogation qui comprend une liste de codes d'identification uniques des emballages (2), et dans laquelle le superviseur de contrôle (6) ou le détecteur (8) est configuré pour sélectionner comme emballages d'échantillon (12) ceux appartenant à la liste présente dans le protocole d'interrogation, et
dans laquelle la procédure de second niveau comprend une étape de vérification, exécutée directement par le superviseur de contrôle (6) ou par le détecteur (8), dans laquelle les emballages d'échantillon (12) identifiés dans chaque sous-groupe (13) sont tous et uniquement ceux présents dans la ou dans une liste du d'un protocole d'interrogation comprenant les codes d'identification uniques des emballages d'échantillon (12) à sélectionner, éventuellement dans laquelle le protocole d'interrogation est stocké dans le superviseur de contrôle (6).

9. Infrastructure de surveillance selon l'une quelconque des revendications 2 à 8, dans laquelle ladite indication fournie par chacun des capteurs de second niveau (5) est lue par le détecteur (8) raccordé de manière à pouvoir communiquer avec le superviseur de contrôle (6) et est transmise à ce dernier, en particulier dans laquelle le détecteur comprend :
- au moins un scanner optique ou une caméra vidéo si l'indication est du type visuel, ou
- au moins un détecteur d'onde radio, si l'indication est du type électromagnétique, ou
- au moins un détecteur acoustique, si l'indication est de type sonore.

10. Infrastructure de surveillance selon l'une quelconque des revendications précédentes, dans laquelle le superviseur de contrôle comprend un ou plusieurs ordinateurs numériques et/ou un ou plusieurs ordinateurs analogiques et dans laquelle le superviseur de contrôle (6) est configuré pour communiquer périodiquement ou à des intervalles réguliers ou suite à une commande ou suite à la détection d'un événement, à un registre distribué (16) de type blockchain raccordé audit superviseur de contrôle (6), au moins l'un ou plusieurs, éventuellement tous, des suivants :
- le code d'identification unique concernant chaque capteur de premier niveau (4),
- la paire de valeurs, formée par une valeur du premier paramètre et instant d'échantillonnage, détectée par chaque capteur de premier niveau (4) durant l'exécution de la procédure de premier niveau,
- le code d'identification unique de chaque capteur de second niveau (5),
- la paire de valeurs, formée par une indication relative au second paramètre et instant de détection, enregistrée par le détecteur (8) pour chaque capteur de second niveau interrogé, et
dans laquelle le superviseur de contrôle (6) est configuré pour certifier quel sous-groupe ou quels sous-groupes (13) présents dans le volume de chargement (3) sont uniquement formés par des produits qui sont conformes à un standard de qualité prédéterminé si, dans un intervalle de temps de référence durant lequel la procédure de premier niveau est exécutée une pluralité de fois, est vérifié comme suit :
- les valeurs du premier paramètre sont conformes au critère prédéterminé d'acceptabilité de premier niveau lors de chaque exécution de la procédure de premier niveau,
ou si
- les valeurs du premier paramètre ne sont pas conformes au critère prédéterminé d'acceptabilité de premier niveau durant au moins une exécution de la procédure de premier niveau, et
- une fois que la procédure de second niveau est exécutée, il est vérifié que lesdits un ou plusieurs sous-groupes (13) sont conformes au critère prédéterminé d'acceptabilité de second niveau ;
éventuellement dans laquelle l'intervalle de temps de référence est égal à la durée d'un transport du chargement d'un point de distribution vers une destination.

11. Procédé mis en œuvre par ordinateur de surveillance d'un chargement de produits périssables, ledit chargement comprenant une multiplicité de produits logés dans des emballages (2) respectifs et placés dans un volume de chargement d'une unité de stockage ou d'un convoyeur, dans lequel :
- le volume de chargement (3) est pourvu d'un nombre prédéterminé de capteurs de premier niveau (4) configurés pour détecter un premier paramètre par rapport à un environnement présent dans le volume de chargement (3) lui-même,
- le volume de chargement (3) comprend une pluralité de sous-groupes (13) de ladite multiplicité de produits présents dans ce volume de chargement, dans lequel chaque sous-groupe (13) comprend une pluralité de produits qui sont, en nombre, une fraction, et en particulier un sous-multiple de ladite multiplicité de produits présents dans le volume de chargement (3),
- chacun des emballages (2) ou des produits logés dans lesdits emballages (2) est associé à au moins un capteur de second niveau (5), chaque capteur de second niveau (5) étant sensible à au moins une altération d'un second paramètre par rapport à chaque emballage (2) ou à un environnement à l'intérieur de chaque emballage (2) ;
dans lequel ledit procédé inclut l'exécution d'une procédure de premier niveau comprenant les étapes suivantes consistant à :
- déterminer les valeurs prises par ledit premier paramètre dans le temps sur la base des détections dudit premier paramètre exécutées par ledit nombre prédéterminé de capteurs de premier niveau (4),
- établir si lesdites valeurs du premier paramètre sont conformes, ou ne sont pas conformes, à un critère prédéterminé d'acceptabilité de premier niveau,
- si les valeurs du premier paramètre sont conformes au critère prédéterminé d'acceptabilité de premier niveau, répéter l'exécution de la procédure de premier niveau ;
et dans lequel ledit procédé inclut une procédure de second niveau qui est exécutée si les valeurs du premier paramètre ne sont pas conformes au critère prédéterminé d'acceptabilité de premier niveau, la procédure de second niveau comprenant les étapes suivantes consistant à :
- établir quels sous-groupes (13) sont conformes à un critère prédéterminé d'acceptabilité de second niveau, en utilisant des indications par rapport à l'altération du second paramètre détectée par au moins une partie des capteurs de second niveau (5) associés aux produits ou aux emballages (2) présents dans chaque sous-groupe respectif.

12. Procédé selon la revendication 11, dans lequel les capteurs de second niveau (5) sont de type différent de ceux des capteurs de premier niveau (4), et/ou
dans lequel le volume de chargement (3) comprend une pluralité de sous-groupes (13) de produits, chaque sous-groupe (13) étant identifiable et distinct des autres sous-groupes, en particulier dans lequel chaque sous-groupe est logé dans une enceinte respective ou placé sur une palette respective, et/ou
dans lequel le procédé comprend l'étape supplémentaire de certification que tous les produits dans le volume de chargement (3) sont conformes à un standard de qualité prédéterminé si, dans un intervalle de temps de référence durant lequel la procédure de premier niveau est exécutée une pluralité de fois, ce qui suit est vérifié :
- les valeurs du premier paramètre sont conformes au critère prédéterminé d'acceptabilité de premier niveau durant chaque exécution de la procédure de premier niveau ;
éventuellement le temps de référence étant égal à la durée d'un transport d'un chargement d'un point de distribution vers une destination.

13. Procédé selon l'une quelconque des deux revendications précédentes, dans lequel le procédé comprend l'étape supplémentaire de certification que les produits dans le volume de chargement (3) satisfont tous un standard de qualité prédéterminé si, dans un intervalle de temps de référence durant lequel la procédure de premier niveau est exécutée une pluralité de fois, ce qui suit est vérifié :
- la procédure de second niveau n'est jamais exécutée ;
et dans lequel le procédé comprend l'étape supplémentaire de certification que tous les produits dans le volume de chargement (3) sont conformes à un standard de qualité prédéterminé si, dans un intervalle de temps de référence durant lequel la procédure de premier niveau est exécutée une pluralité de fois, ce qui suit se produit :
- si la procédure de second niveau est exécutée, il est vérifié que tous les sous-groupes sont conformes au critère prédéterminé d'acceptabilité de second niveau, et
dans lequel la procédure de second niveau inclut l'étape additionnelle de signalement des sous-groupes qui ne sont pas conformes au critère prédéterminé d'acceptabilité de second niveau.

14. Procédé selon l'une quelconque des revendications 11 à 13, dans lequel le premier paramètre est détecté à la position à l'intérieur du volume de chargement dans lequel chaque capteur de premier niveau (4) fonctionne ; en particulier dans lequel une pluralité de capteurs de premier niveau (4) sont distribués, éventuellement uniformément distribués, dans le volume de chargement, et
dans lequel le critère prédéterminé d'acceptabilité de premier niveau est défini par une plage de températures, ou par une plage de valeurs d'acceptabilité de paramètre comme une fonction de température ou de variation de température ;
ou dans lequel le critère prédéterminé d'acceptabilité de premier niveau est défini par une plage de concentrations ou une variation de concentration d'un gaz ou d'un mélange de gaz prédéterminé, et
dans lequel le second paramètre est la température, ou un paramètre qui dépend de la température, de chaque emballage (2) ou d'un environnement à l'intérieur de chaque emballage ; ou le second paramètre est la température d'une surface externe de l'emballage (2) ; ou le second paramètre est la température à l'intérieur de chaque emballage (2) ; ou le second paramètre est la concentration ou la variation de concentration d'un gaz prédéterminé à l'intérieur de l'emballage (2) ; ou le second paramètre est la concentration ou la variation de concentration d'une substance prédéterminée à l'intérieur de l'emballage ; ou le second paramètre est le degré d'acidité présent à l'intérieur de l'emballage (2) ou la variation du degré d'acidité dans chaque emballage (2).

15. Procédé selon l'une quelconque des revendications 11 à 14, dans lequel chacun des capteurs de second niveau (5) fournit que le critère prédéterminé d'acceptabilité de second niveau est considéré comme étant satisfait pour un sous-groupe (13) donné uniquement si l'indication, éventuellement visuelle, fournie par chacun des capteurs de second niveau (5) utilisés dans la procédure de second niveau pour ce sous-groupe est conforme à un test respectif de vérification, et
dans lequel l'étape de la procédure de second niveau de détermination des sous-groupes qui sont conformes à un critère prédéterminé d'acceptabilité de second niveau inclut les étapes consistant à :
- identifier dans chaque sous-groupe (13) un nombre prédéterminé d'emballages d'échantillon (12), lesdits emballages d'échantillon (12) étant inférieurs en nombre au nombre total d'emballages (2) dans chaque sous-groupe (13), en particulier où lesdits emballages d'échantillon (12) sont inférieurs à la moitié du nombre d'emballages (2) dans chaque sous-groupe (13),
- établir qu'un sous-groupe satisfait le critère prédéterminé d'acceptabilité de second niveau exclusivement en utilisant les capteurs de second niveau (5) associés aux emballages d'échantillon (12), ou aux produits contenus dans les emballages d'échantillon (12), présents dans le sous-groupe (13) respectif, et
dans lequel les emballages d'échantillon (12) de chaque sous-groupe (13) sont placés dans une zone périphérique externe du sous-groupe,
dans lequel chaque sous-groupe présente des emballages qui sont placés côte à côte et surimposés l'un sur l'autre pour former un cube idéal ou un parallélogramme idéal, et dans lequel chacun des emballages d'échantillon (12) est placé au niveau d'un bord ou d'un sommet respectif du cube idéal ou du parallélogramme ; éventuellement les emballages d'échantillon (12) sont de deux ou plusieurs en nombre pour chaque sous-groupe (13).

16. Procédé selon l'une quelconque des revendications 11 à 15, dans lequel le procédé comprend l'étape supplémentaire de certification qu'un ou plusieurs sous-groupes (13) présents dans le volume de chargement sont exclusivement constitués de produits se conformant à un standard de qualité prédéterminé si, dans un intervalle de temps de référence durant lequel la procédure de premier niveau est exécutée une pluralité de fois, ce qui suit se produit :
- les valeurs du premier paramètre sont conformes au critère prédéterminé d'acceptabilité de premier niveau chaque fois que la procédure de premier niveau est exécutée, et
dans lequel le procédé comprend l'étape supplémentaire de certification qu'un ou plusieurs sous-groupes (13) présents dans le volume de chargement sont exclusivement constitués de produits se conformant à un standard de qualité prédéterminé si, dans un intervalle de temps de référence durant lequel la procédure de premier niveau est exécutée une pluralité de fois, ce qui suit se produit :
- les valeurs du premier paramètre ne sont pas conformes au critère prédéterminé d'acceptabilité de premier niveau durant au moins une exécution de la procédure de premier niveau, et
- lorsque la procédure de second niveau est exécutée, il est vérifié que lesdits un ou plusieurs sous-groupes (13) sont conformes au critère prédéterminé d'acceptabilité de second niveau.

17. Procédé selon l'une quelconque des revendications 11 à 16, dans lequel le procédé est exécuté par l'infrastructure selon l'une quelconque des revendications 1 à 10.

18. Progiciel comprenant des instructions qui lorsqu'elles sont exécutées par une unité de contrôle programmable configurent ladite unité de contrôle programmable pour exécuter un procédé selon l'une quelconque des revendications 11 à 17,
éventuellement dans lequel lesdites instructions sont stockées dans une mémoire comprenant une mémoire disque optique ou magnétique ou une mémoire type EPROM ou une mémoire volatile ;
ou dans lequel lesdites instructions sont portées par un signal électromagnétique, plus facultativement dans un paquet de données transmis par l'intermédiaire d'Internet.
